# EUROPEAN PATENT SPECIFICATION

(11) **EP 4 084 691 B1**
(45) Date of publication and mention of the grant of the patent: **10.12.2025**
(21) Application number: 20845716.8
(22) Date of filing: 21.12.2020
(51) Int. Cl.: A61B 5/282, A61B 5/363, A61B 5/00, A61N 1/362, A61B 5/361, A61B 5/364

(54) **WEARABLE MEDICAL DEVICE WITH REMOVABLE SUPPORT GARMENT**
TRAGBARE MEDIZINISCHE VORRICHTUNG MIT ABNEHMBARER STÜTZKLEIDUNG
DISPOSITIF MÉDICAL POUVANT ÊTRE PORTÉ AVEC VÊTEMENT DE SUPPORT AMOVIBLE

(30) Priority: 30.12.2019 US 201962954886 P
(43) Date of publication of application: 09.11.2022
(60) Divisional application: 25182821.6
(73) Proprietor: Zoll Medical Corporation, Chelmsford Massachusetts 01824-4105 (US)
(72) Inventor: FREEMAN, Gary, A., Waltham, Massachusetts 02453 (US)
(74) Representative: Potter Clarkson
(86) International application number: PCT/US2020/066412
(87) International publication number: WO 2021/138121

(56) References cited:
- US-A- 5 313 952
- US-A1- 2008 033 495
- US-A1- 2012 283 794
- US-A1- 2016 256 104
- US-A1- 2017 056 650
- US-A1- 2019 022 400
- US-A1- 2019 282 821

## Description

### BACKGROUND

The present disclosure is directed to wearable cardiac monitoring and treatment devices

A patient suffering from heart failure experiences symptoms caused by a weak or damaged heart contracting inefficiently and failing to pump effectively to circulate oxygenated blood through the body. A heart may be weakened by, for example, abnormal heart rhythms (e.g., heart arrhythmias), high blood pressure, coronary artery disease, myocardial infarction, and myocarditis.

Left untreated, heart failure could lead to certain life-threatening arrhythmias. Both atrial and ventricular arrhythmias are common in patients with heart failure. One of the deadliest cardiac arrhythmias is ventricular fibrillation, which occurs when normal, regular electrical impulses are replaced by irregular and rapid impulses, causing the heart muscle to stop normal contractions. Because the victim has no perceptible warning of the impending fibrillation, death often occurs before the necessary medical assistance can arrive. Other cardiac arrhythmias can include excessively slow heart rates known as bradycardia or excessively fast heart rates known as tachycardia.

Cardiac arrest can occur when various arrhythmias of the heart, such as ventricular fibrillation, ventricular tachycardia, pulseless electrical activity (PEA), and asystole (heart stops all electrical activity), result in the heart providing insufficient levels of blood flow to the brain and other vital organs for supporting life. It is generally useful to monitor heart failure patients in order to assess heart failure symptoms early and provide interventional therapies as soon as possible.

Wearable cardiac monitoring and treatment devices are provided to monitor for such arrhythmias and provide a treatment when a life-threatening arrhythmia is detected. Such devices are worn by the patient continuously to provide constant protection as the patient goes about his or her daily routine. As such, the devices need to be designed to be comfortable, secure, and easy to use.

US2017/056650A1 relates to an electrode patch for use with an external medical device. US2008/033495A1 relates to an external defibrillator having a battery; a capacitor electrically communicable with the battery; at least two electrodes electrically communicable with the capacitor and with the skin of a patient; a controller configured to charge the capacitor from the battery and to discharge the capacitor through the electrodes; and a support supporting the battery, capacitor, electrodes and controller in a deployment configuration, the defibrillator having a maximum weight per unit area in the deployment configuration of 0.1 lb/in² and/or a maximum thickness of 1 inch.

### SUMMARY

The invention is defined by claim 1. The following examples are also disclosed. In one example, a patient-worn arrhythmia monitoring and treatment device includes at least two pads, a controller coupled to one of the at least two pads, and a removable garment configured to be worn about the torso of the patient to immobilize on the torso the one of the at least two pads to which the controller is coupled. The at least two pads include an adhesive layer configured to affix each one of the at least two pads to skin on a torso of a patient, at least one of a pair of sensing electrodes disposed on each one of the at least two pads, and at least one of a pair of therapy electrodes disposed on each one of the at least two pads. The pair of sensing electrodes are configured to sense surface ECG activity of the patient, and the pair of therapy electrodes configured to deliver one or more therapeutic pulses to the patient. The controller is configured to be coupled to one of the at least two pads and in communication with the pairs of sensing and therapy electrodes. The controller is configured to monitor for cardiac arrhythmias based on the sensed surface ECG activity of the patient and cause the delivery of the one or more therapeutic pulses to the patient.

Implementations of the device may include one or more of the following features.

In examples, the least two pads are configured for continuous wear for durations of at least one of a day, one week, two weeks, a month, six months, and one year. In examples, each one of the at least two pads includes a breathable hydrogel layer disposed between the patient's skin and each of the pairs of sensing and therapy electrodes to facilitate the continuous wear. In examples, the adhesive layer includes a breathable adhesive material to facilitate the continuous wear. In examples, each one of the at least two pads includes waterproof or water-resistant material to prevent ingress of liquid between the patient's skin and each one of the at least two pads to facilitate the continuous wear at least during periods of bathing or showering.

In examples, the removable garment is configured for selective wear during certain activities of finite duration including at least one of athletic activities, showering, bathing, and sleeping. In examples, the removable garment includes a retention feature configured to facilitate the selective wear during certain activities by allowing separation of the removable garment from the one of the at least two pads to which the controller is coupled.

In examples, a first of the at least two pads has a first weight and a second of the at least two pads has a second weight, wherein the first weight is greater than the second weight. The removable garment can be configured to at least partially support the weight of the first of the at least two pads. In examples, the first of the at least two pads weighs between 1kg-2.5kg and the second of the at least two pads weighs between 0.5kg-1kg, and the first of the at least two pads is the one of the at least two pads to which the controller is coupled. In examples, the controller includes one or more of capacitors, batteries, processors, printed circuit boards, ECG acquisition and conditioning circuitry, and high-voltage therapy control circuitry disposed within the housing.

In examples, the removable garment is at least one of a shoulder strap, a vest, a shirt, a belt, a harness, a bandeau, and a sash.

In examples, the removable garment is a compression garment configured to be worn over at least one of the at least two pads and apply a compression force to maintain contact of the at least one of the at least two pads with the torso.

In examples, the removable garment includes a closure mechanism including at least one of a ratchet strap, an adjustable buckle, an extendable and moveable hook and loop fastener strip, a tie, a snap, and a button.

In examples, immobilizing the one of the at least two pads to which the controller is coupled includes at least partially supporting a weight of the one of the at least two pads to which the controller is coupled. The controller can be disposed within a housing that is coupled to one of the at least two pads, and the removable garment further includes a retention feature for engaging with the housing. In examples, the retention feature includes a hole sized and shaped to accommodate the housing when inserted through the hole. In examples, the retention feature includes a fastener including at least one of hook and loop fastener strip, a tie, a snap, and a button. In examples, the retention feature includes an interlock disposed on an interior surface of the removable garment, the interlock configured to engage a corresponding mating portion disposed on an exterior surface of the housing. The retention feature can include a non-slip surface disposed on an interior surface of the removable garment such that the removable garment remains stationary relative to the housing. The retention feature can include a conforming cup formed as non-removable portion of the removable garment such that the removable garment remains stationary relative to the housing when the housing is received in the conforming cup. The controller can include one or more of capacitors, batteries, processors, printed circuit boards, ECG acquisition and conditioning circuitry, and high-voltage therapy control circuitry disposed within the housing.

In one example, a patient-worn arrhythmia monitoring and treatment device, includes a removable wearable support configured to at least partially support a weight of the patient-worn arrhythmia monitoring and treatment device. The removable wearable support includes a fabric encircling a torso of a patient and an engagement surface disposed on one side of the fabric. The engagement surface is configured to engage immovably with at least one of two pads configured to be affixed to an upper torso of the patient. The at least one of two pads includes an adhesive layer configured to affix the at least one of two pads to skin on the upper torso of the patient, at least one of a pair of sensing electrodes disposed on the at least one of two pads, the pair of sensing electrodes configured to sense surface ECG activity of the patient, at least one of a pair of therapy electrodes disposed on the at least one of two pads, the pair of therapy electrodes configured to deliver one or more therapeutic pulses to the patient, a housing coupled to the at least one of two pads, the housing configured to engage with the engagement surface of the removable wearable support, and at least one processor disposed in the housing. The at least one processor is in communication with the pairs of sensing and therapy electrodes and configured to monitor for cardiac arrhythmias based on the sensed surface ECG activity of the patient and cause the delivery of the one or more therapeutic pulses to the patient.

Implementations of the device may include one or more of the following features.

In examples, the at least two pads are configured for continuous wear for durations of at least one of a day, one week, two weeks, a month, six months, and one year.

In examples, each one of the at least two pads includes a breathable hydrogel layer disposed between the patient's skin and each of the pairs of sensing and therapy electrodes to facilitate the continuous wear.

In examples, the adhesive layer includes a breathable adhesive material to facilitate the continuous wear.

In examples, each one of the at least two pads includes waterproof or water-resistant material to prevent ingress of liquid between the patient's skin and each one of the at least two pads to facilitate the continuous wear at least during periods of bathing or showering.

In examples, the removable wearable support is configured for selective wear during certain activities of finite duration including at least one of athletic activities, showering, bathing, and sleeping.

In examples, the removable wearable support includes a retention feature configured to facilitate the selective wear during certain activities by allowing separation of the removable wearable support from the one of the at least two pads to which the controller is coupled.

In examples, a first of the at least two pads has a first weight and a second of the at least two pads has a second weight, wherein the first weight is greater than the second weight. The removable wearable support can be configured to at least partially support the weight of the first of the at least two pads. In examples, the first of the at least two pads weighs between 1kg-2.5kg and the second of the at least two pads weighs between 0.5kg-1kg, and the first of the at least two pads is the one of the at least two pads to which the controller is coupled. In examples, the controller includes one or more of capacitors, batteries, processors, printed circuit boards, ECG acquisition and conditioning circuitry, and high-voltage therapy control circuitry disposed within the housing.

In examples, the removable wearable support is at least one of a shoulder strap, a vest, a shirt, a belt, a harness, a bandeau, and a sash. The removable wearable support can be a compression garment configured to be worn over at least one of the at least two pads and apply a compression force to maintain contact of the at least one of the at least two pads with the torso.

In examples, the removable wearable support includes a closure mechanism including at least one of a ratchet strap, an adjustable buckle, an extendable and moveable hook and loop fastener strip, a tie, a snap, and a button.

In examples, immobilizing the one of the at least two pads to which the controller is coupled includes at least partially supporting a weight of the one of the at least two pads to which the controller is coupled. The controller can be disposed within a housing that is coupled to one of the at least two pads, and the removable wearable support can include a retention feature for engaging with the housing. In examples, the retention feature includes a hole sized and shaped to accommodate the housing when inserted through the hole. In examples, the retention feature includes a fastener including at least one of hook and loop fastener strip, a tie, a snap, and a button. In examples, the retention feature includes an interlock disposed on an interior surface of the removable wearable support, the interlock being configured to engage a corresponding mating portion disposed on an exterior surface of the housing. In examples, the retention feature includes a non-slip surface disposed on an interior surface of the removable wearable support such that the removable wearable support remains stationary relative to the housing. In examples, the retention feature includes a conforming cup formed as non-removable portion of the removable wearable support such that the removable wearable support remains stationary relative to the housing when the housing is received in the conforming cup. In examples, the controller includes one or more of capacitors, batteries, processors, printed circuit boards, ECG acquisition and conditioning circuitry, and high-voltage therapy control circuitry disposed within the housing.

### BRIEF DESCRIPTION OF THE DRAWINGS

FIG. 1 depicts a schematic of an example patient-worn arrhythmia monitoring and treatment device including a wearable garment.
FIGS. 2A depicts a schematic of an example patient-worn arrhythmia monitoring and treatment device including anterior mounted first and second assemblies.
FIGS. 2B depicts a schematic of an example adhesively coupled patient-worn arrhythmia monitoring and treatment device including one posterior mounted first assembly and an anterior mounted second assembly.
FIGS. 2C depicts a schematic of an example of a portion of the adhesively coupled patient-worn arrhythmia monitoring and treatment device of FIGS. 2A and 2B.
FIG. 3 depicts a side cross-section schematic of an example patient-worn arrhythmia monitoring and treatment device.
FIG. 4 depicts an example schematic of electrically connected components of the patient-worn arrhythmia monitoring and treatment device of FIG. 3.
FIG. 5 depicts a side cross-section schematic of an example adhesive pad assembly of an example patient-worn arrhythmia monitoring and treatment device.
FIG. 6A depicts a schematic of an example patient-worn arrhythmia monitoring and treatment device including a removable garment and adhesively coupled portions in wireless communication.
FIG. 6B depicts a schematic of an example patient-worn arrhythmia monitoring and treatment device including a removable garment and adhesively coupled portions in wireless communication.
FIG. 6C depicts a schematic of an example patient-worn arrhythmia monitoring and treatment device including a removable garment and at least one adhesively coupled portion disposed between the removable garment and a torso of the patient.
FIG. 6D depicts a schematic of an example patient-worn arrhythmia monitoring and treatment device including a removable garment and at least one adhesively coupled portion disposed between the removable garment and a torso of the patient.
FIG. 7 depicts a schematic of an example patient-worn arrhythmia monitoring and treatment device including two separate removable garments and adhesively coupled portions disposed between the removable garment and a torso of the patient.
FIG. 8A depicts a schematic of an example removable wearable garment of patient-worn arrhythmia monitoring and treatment device including a retention feature for accommodating housing of the wearable cardiac monitoring and treatment device therethrough.
FIG. 8B depicts a schematic of an example removable wearable garment of a patient-worn arrhythmia monitoring and treatment device including a retention feature for engaging a surface of a housing wearable cardiac monitoring and treatment device.
FIG. 8C depicts a schematic of an example removable wearable garment of a patient-worn arrhythmia monitoring and treatment device including a retention feature for receiving a housing of a wearable cardiac monitoring and treatment device in a form-fitted engagement.
FIG. 9 depicts a schematic of an example removable wearable garment of a patient-worn arrhythmia monitoring and treatment device including an optionally-worn auxiliary strap.
FIG. 10 is a schematic of an example method of using a patient-worn arrhythmia monitoring and treatment device.
FIG. 11 depicts a schematic diagram of an embodiment of a patient-worn arrhythmia monitoring and treatment device.
FIG. 12 depicts a schematic diagram of an embodiment of electrical components of a patient-worn arrhythmia monitoring and treatment device.

### DETAILED DESCRIPTION

This disclosure relates to a patient-worn arrhythmia monitoring and treatment device that detects one or more treatable arrhythmias based on physiological signals from a patient. The treatable arrhythmias include those that may be treated by defibrillation pulses, such as ventricular fibrillation (VF) and shockable ventricular tachycardia (VT), or by one or more pacing pulses, such as bradycardia, tachycardia, and asystole. Patients prescribed with such life-saving devices need to be able to wear them continuously through daily activities to ensure near constant protection against life-threatening cardiac arrhythmia conditions over extended periods of time. Accordingly, the devices herein provide improved ergonomics and physiological benefits that promote better voluntary use within device use guidelines than conventional devices. A wearable medical device as disclosed herein is adhesively coupled to a patient and monitors the patient's physiological conditions, e.g., cardiac signals, respiratory parameters, and patient activity, and delivers potentially life-saving treatment to the patient. Embodiments of patient-worn adhesively coupled cardiac monitoring and treatment devices can include one or more removable garments or wearable supports for further securing and immobilizing against the torso one or more components adhesively coupled to the torso of a patient during certain periods of activity with additional securement is preferred.

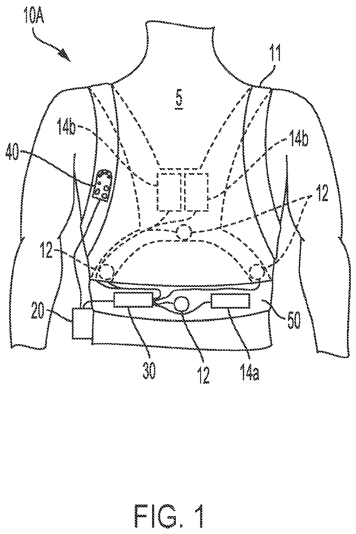

Referring to FIG. 1, an example wearable cardiac device 10A is shown. Device 10A is a wearable cardioverter defibrillator (WCD) such as the LifeVest^{®} WCD from ZOLL Medical Corporation of Chelmsford, MA. In implementations, WCD 10A includes a garment 11, one or more physiological sensors 12 (e.g., ECG electrodes, heart rate sensors, vibrational sensors, and/or other physiological sensors), one or more therapy electrodes 14a and 14b (together 14), a medical device controller 20, a connection pod 30, a patient interface pod 40, and a belt 50 about the patient's torso to support one or more components. At least some of the components of WCD 10A may be affixed to the garment 11 (or in alternative implementations, permanently integrated into the garment 11), which can be worn about the patient's torso 5. The medical device controller 20 is operatively coupled to the physiological sensors 12 affixed to or permanently integrated into the garment 11. U.S. Patent No. 8,983,597, titled "MEDICAL MONITORING AND TREATMENT DEVICE WITH EXTERNAL PACING," issued on March 17, 2015 (hereinafter the "'597 Patent"), describes an example wearable cardioverter defibrillator (WCD) device.

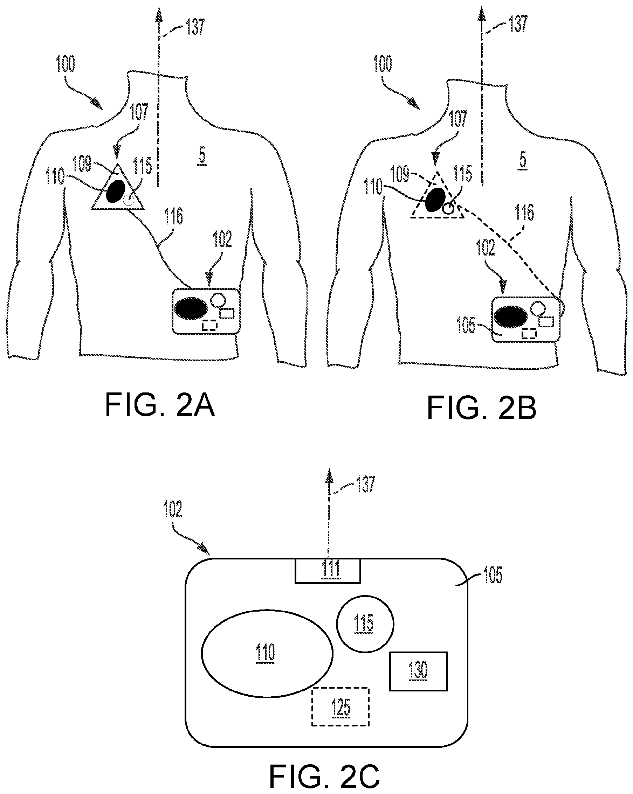

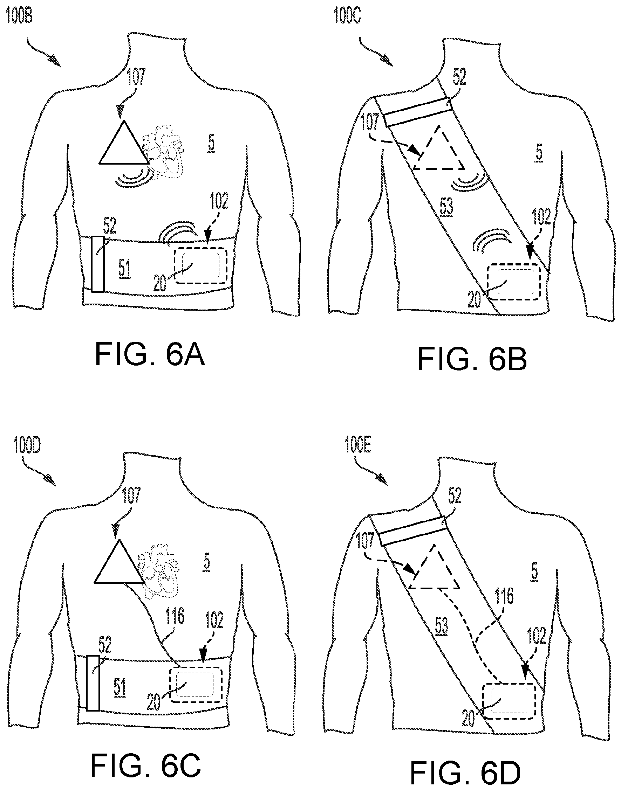

In embodiments according to this disclosure, such as that of FIGS. 2A-C and 6A-D, instead of a garment to which treatment and monitoring components are affixed, one or more of the cardiac treatment and monitoring components can be adhesively affixed to the torso of the patient, and supported by a removable garment or wearable support. In this regard, in examples described herein, a patient-worn arrhythmia monitoring and treatment device can include at least two separable portions. A first portion includes adhesively affixed components such as electrodes and housings comprising the monitoring and treatment circuit components as described in detail to follow. A second portion includes a removable garment or wearable support to be worn about the torso over the adhesively affixed components for certain durations of time, for example while a patient exercises, sleeps, or bathes.

In examples, the removable wearable support can be in the form of a shoulder strap, a vest, a belt, a harness, a bandeau, a sash or a combination of one or more of such forms. In implementations, the wearable support can be fitted to the body as a lightweight stretchable support garment, or other structure for supporting heavier components of the device. In one example, the wearable support may be a belt 50 or sash 53, as shown in FIGS. 6A through 6D. The belt 50 or sash 53 can be configured to immobilize at least the heavy components of the device that are adhesively attached to the torso 5 of the patient so that they do not peel off the torso during certain activities, e.g. exercise, bathing, and sleeping.

For example, some patients may desire to maintain an active lifestyle during a prescribed duration of wear of the monitoring and treatment device. A patient may participate in regular physical exercise, such as walking, jogging, aerobic or other physical activity involving vigorous, sudden, jerky, repetitive and/or continuous movements. In examples, such activities may include stretching, jumping, rotational, and/or lunging motions. With each such stride, step, bounce, leap and/or twist, the adhesively affixed device undergoes the physical impacts of at least vibrational, gravitational, and shear forces.

In implementations herein, the removable garment is a breathable and stretchable garment, for example, that presses the adhesively affixed device against the skin of the patent to reduce or eliminate motion of the device relative to the torso during physical exercise. For example, the removable garment can reduce or eliminate moment and shear forces and prevent the device from delaminating from the skin of the patient. Maintaining a stationary position of the device, and sensors thereon, on the skin of the patient during periods of activity or significant physical movement prevents signal noise associated with the motion, thereby reducing or eliminating false positive detection of cardiac arrhythmias.

In another example, because patients are encouraged to wear such monitoring and treatment devices continuously, the removable garment can provide added assistance during instances of showering or bathing. The removable garment can hold the adhesive device against the skin of the patient, maintaining the adhesive seal intact against the torso. In implementations, in addition to providing a compression force to keep the adhesive intact, the removable garment can be waterproof at least in a region covering the adhesive device such that the device is shielded from the ingress of liquids and solvents that might otherwise deteriorate the adhesive sealing the device to the torso and/or infiltrate the device. A patient can feel reassured that the electronic device adhered to the torso is further protected from liquid ingress by the removable garment.

In still some cases, implementations herein promote patient comfort during periods of sleep or resting while lying down. A patient can wear the removable garment during their hours of sleep or rest to prevent delamination of the adhesive device and potential device alarms associated with loss of contact of one or more monitoring sensors with the torso. Additionally, the removable garment can include one or more compression and/or padded regions for comfort while the patient is prone and lying on a mattress. A removable garment including on or more regions of padding can assist with accommodating the adhesive device protruding from the torso and facilitating repositioning and rolling over on a mattress without dislodging the device from the torso. A garment worn over an adhesively attached device can protect any protrusions and/or edges of the adhesive device from getting caught on the patient's clothing and bedding (e.g., comforters, covers and sheets) during patient movement. Such a garment can help ease concerns of accidentally peeling off the device while rolling over during sleep. In some implementations, the entire removable garment can includes a comfort compression or padded layer to prevent discomfort during many hours of continuous wear. Such a compression layer can prevent formation of bedsores while the patient is sleeping and wearing the removable garment.

Because these devices require continuous operation and wear by patients to which they are prescribed, advantages of the implementations herein include use of comfortable, non-irritating, biocompatible adhesive and construction materials, and features designed to enhance patient use. Such features include, for example, providing a removable garment to secure adhesively mounted components to the torso during selective periods of activity. The removable garment supports some of the weight of the adhesively mounted components during the periods of activity rendering the device more comfortable to wear. Such features also include device ergonomics, weight and/or distribution of the weight of the components, overall device shape, and inconspicuous appearance when worn under output garments, among others.

The example devices described herein are prescribed to be worn continuously and typically for a prescribed duration of time. For example, the prescribed duration can be a duration for which a patient is instructed by a caregiver to wear the device in compliance with device use instructions. The prescribed duration may be for a short period of time until a follow up medical appointment (e.g., 1 hour to about 24 hours, 1 day to about 14 days, or 14 days to about one month), or a longer period of time (e.g., 1 month to about 3 months, 3 months to about 6 months, or 6 months to about a year) during which diagnostics information about the patient is being collected even as the patient is being protected against cardiac arrhythmias. The prescribed use is uninterrupted until a physician or other caregiver provides a specific prescription to the patient to stop using the wearable medical device. For example, the wearable medical device can be prescribed for use by a patient for a period of at least one week. In an example, the wearable medical device can be prescribed for use by a patient for a period of at least 30 days. In an example, the wearable medical device can be prescribed for use by a patient for a period of at least one month. In an example, the wearable medical device can be prescribed for use by a patient for a period of at least two months. In an example, the wearable medical device can be prescribed for use by a patient for a period of at least three months. In an example, the wearable medical device can be prescribed for use by a patient for a period of at least six months. In an example, the wearable medical device can be prescribed for use by a patient for an extended period of at least one year.

A sudden cardiac arrest or other arrhythmia condition can strike at any time and with little warning. Accordingly, every patient is encouraged to follow device use guidelines, including wearing the device at all times during a prescribed duration, including while showering, sleeping, or while performing exercise activities. To improve patient use of the device, the devices described herein are lightweight, comfortable, and compact so that they may be concealed under the patient's clothing. Moreover, the devices are configured to allow for uncomplicated application and adherence to the skin of the body of the patient. In some implementations described herein, the devices include various features that promote comfort while continuing to protect the patient from adverse physiological reactions or cardiac events. These features can be tailored in accordance with patient comfort preference and can include durable adherence, ease of application and removal, and inconspicuous appearance.

The devices include biocompatible adhesives, such as pressure-sensitive adhesives having tack, adhesion, and cohesion properties suitable for use with a medical device applied to skin for both short and long-term durations. These pressure sensitive adhesives can include polymers such as acrylics, rubbers, silicones, and polyurethanes having a high initial tack for adhering to skin. These pressure sensitive adhesives also maintain adhesion during showering or while a patient is perspiring. The adhesives also enable removal without leaving behind uncomfortable residue. For example, such an adhesive can be a rubber blended with a tackifier.

In any of the previously presented or foregoing examples, the devices herein include low skin-irritation adhesives. In embodiments, the device may be worn continuously by a patient for a long-term duration (e.g., duration of at least one week, at least 30 days, at least one month, at least two months, at least three months, at least six months, and at least one year) without the patient experiencing significant skin irritation. For example, a measure of skin irritation can be based on skin irritation grading of one or more as set forth in Table C.1 of Annex C of American National Standard ANSI/AAMI/ISO 10993-10:2010, reproduced here in the entirety:

**Table 1.**

| Table C.1 - Human Skin irritation test, grading scale | |
|---|---|
| **Description of response** | **Grading** |
| No reaction | 0 |
| Weakly positive reaction (usually characterized by mild erythema and/or dryness across most of the treatment site) | 1 |
| Moderately positive reaction (usually distinct erythema or dryness, possibly spreading beyond the treatment site) | 2 |
| Strongly positive reaction (strong and often spreading erythema with oedema and/or eschar formation) | 3 |

The skin irritation grading of one represents a weakly positive reaction usually characterized by mild erythema and/or dryness across most of the treatment site. In one implementation, a measure of skin irritation can be determined by testing on human subjects in accordance with the method set forth in American National Standard ANSI/AAMI/ISO 10993-10:2010, by applying sample patches of the adhesive device to treatment sites for up to four hours, and, in the absence of skin irritation, subsequently applying sample patches to treatment sites for up to 24 hours. The treatment sites are examined for signs of skin irritation, and the responses are scored immediately after patch removal and at time intervals of (1 ± 0.1) h to (2 ± 1) h, (24 ± 2) h, (48 ± 2) h and (72 ± 2) h after patch removal. In another implementation, a patient may wear the adhesive device as instructed for a duration of (24± 2) hours, and if the patient's skin shows no reaction at the end of this duration, the adhesive device is rated as a skin irritation grading of zero.

In addition to biocompatible adhesives, such short term and long-term wear devices include a plurality of sensing electrodes that are disposed on the patient's body and configured to monitor cardiac signals such as electrocardiogram (ECG) signals. The devices, therefore, determine an appropriate treatment for the patient based on the detected cardiac signals and/or other physiological parameters prior to delivering a therapy to the patient. The devices then cause one or more therapeutic shocks, for example, defibrillating and/or pacing shocks, to be delivered to the body of the patient. The wearable medical device includes a plurality of therapy electrodes, at least one of which is integrated within a contoured pad as described in detail herein. The plurality of therapy electrodes are disposed on the patient's body and configured to deliver the therapeutic shocks. In some implementations, the devices can also be configured to allow a patient to report his/her symptoms including one or more skipped beat(s), shortness of breath, light headedness, racing heart, fatigue, fainting, and chest discomfort. Device implementations and example features are disclosed herein to improve the comfort and durability of such a wearable medical device, especially during periods of activity.

In implementations, the devices include one or more contoured pads configured to be adhesively secured to the torso of the patient. One or more energy storage units are operably connected to a therapy delivery circuit. The energy storage units as well as a therapy delivery circuit are housed within at least one housing configured to form a watertight seal with the contoured pad. In some implementations, a plurality of housings can be disposed on a plurality of segments of the contoured pad. Each of the plurality of housings can include different portions of the device circuitry, such as, ECG acquisition and conditioning circuit(s), therapy delivery circuit(s), energy storage unit(s), processor(s), power source(s) and the like. The energy storage units are configured to store energy for at least one therapeutic pulse (e.g., a defibrillation pulse). The therapy delivery circuit is configured to cause the delivery of the at least one therapeutic pulse via the plurality of therapy electrodes. In implementations, the energy storage units are electrically coupled to the plurality of therapy electrodes (e.g., by a printed circuit board trace, a flex circuit, or a direct contact connection).

As described above, implementations of the wearable medical device described herein are capable of continuous use by patients during either the short term or long-term wear duration. Such continuous use can be substantially continuous or nearly continuous in nature. During substantially continuous or nearly continuous use, the wearable medical device may be continuously used except for sporadic periods during which the use temporarily ceases (e.g., while the patient is refit with a new and/or a different device, while the battery is charged and/or changed, etc.). Such substantially continuous or nearly continuous use as described herein may nonetheless qualify as continuous use. For example, the continuous use can include continuous wear or attachment of the wearable medical device to the patient. In implementations, one or more of the electrodes are continuously attached to the patient as described herein during both periods of monitoring and periods when the device may not be monitoring the patient but is otherwise still worn by or otherwise attached to the patient. Continuous use can include continuously monitoring the patient while the patient is wearing the device for cardiac-related information (e.g., electrocardiogram (ECG) information, including arrhythmia information, cardiac vibrations, etc.) and/or non-cardiac information (e.g., blood oxygen, the patient's temperature, glucose levels, tissue fluid levels, and/or pulmonary vibrations). For example, the wearable medical device can carry out its continuous monitoring and/or recording in periodic or aperiodic time intervals or times (e.g., every few minutes, every few hours, once a day, once a week, or other interval set by a technician or prescribed by a caregiver). Alternatively or additionally, the monitoring and/or recording during intervals or times can be triggered by a user action or another event.

As noted above, the wearable medical device can be configured to monitor other physiologic parameters of the patient in addition to cardiac related parameters. For example, the wearable medical device can be configured to monitor, for example, pulmonary vibrations (e.g., using microphones and/or accelerometers), breath vibrations, sleep related parameters (e.g., snoring, sleep apnea), and tissue fluids (e.g., using radio-frequency transmitters and sensors), among others.

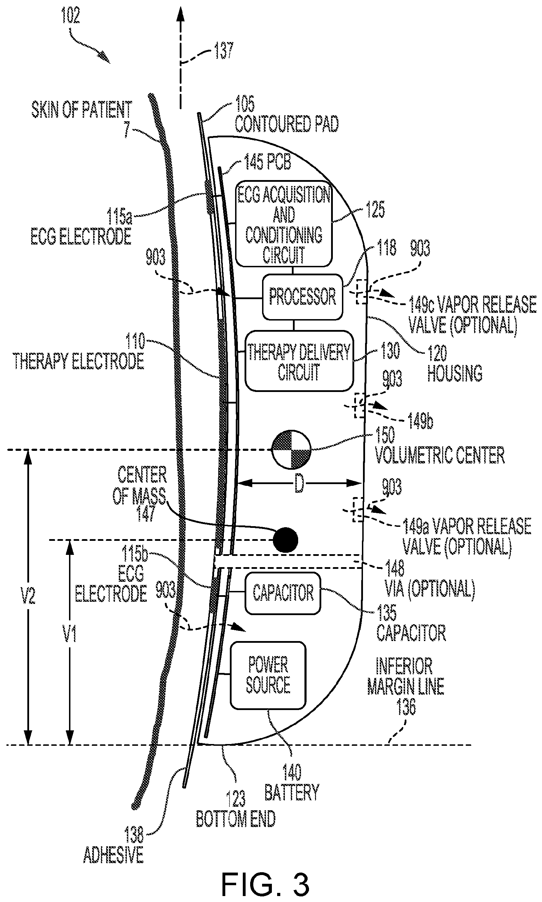

An example adhesively coupled monitoring and treatment device 100 is shown in FIGS. 2A-B. As shown, the device 100 is external, ambulatory, and adhesively coupled to a patient. The medical device 100 is an external or non-invasive medical device, which, for example, is located external to the body of the patient and configured to provide transcutaneous therapy to the body. The device 100 is an ambulatory medical device, which, for example, is capable of and designed for moving with the patient as the patient goes about his or her daily routine. The device 100 includes a first assembly 102 that includes a pad 105 configured to be adhesively coupled to a torso 5 of a patient. In implementations, a plurality of therapy electrodes and/or a plurality of ECG sensing electrodes can be integrated with the pad. Further, as shown in FIG. 3 the device 100 can include a housing 120 configured to form a watertight seal with the pad 105. In certain implementations, the housing 120 can extend between around 1 cm and 5 cm from a surface of the pad. The housing can include at least an ECG acquisition and conditioning circuit, a therapy delivery circuit, and a processor. For example, the processor can analyze the ECG signal of the patient received and conditioned via the ECG acquisition and conditioning circuit and detect one or more treatable arrhythmias. The processor can cause the therapy delivery circuit to deliver at least one defibrillation pulse to the patient on detecting the one or more treatable arrhythmias. The device 100 includes components with particular physical dimensions, weights, and functional properties that in combination cause the overall weight of the device 100 to be in a range of 250 grams to 2,500 grams while enabling the device 100 to function as a monitoring and treatment device.

In examples, the first assembly 102 can be coupled to a second assembly 107 that includes a second, different pad 109 as shown in FIGS. 2A-B. For example, the second assembly 107 can be configured to be located at an upper right anterior position of the patient's torso 5 as shown in FIG. 2A. In other examples, the second assembly 107 can be configured to be located at an upper posterior position of the patient's torso 5, such as the upper right posterior position, as shown in FIG. 2B. Although the implementations of FIGS. 2A and 2B depict a substantially rectangular shaped first assembly 102 and a triangle shaped second assembly, the shapes of the first and second assemblies can be any shape including, for example, a polygon, a square, a circle, an oval, an octagon, a trefoil, a trapezoid, a polygon, or a non-polygon shape custom-tailored to a patient's contours and/or preferences. In implementations, the second assembly 107 can include one or more of the components and/or materials described with regard to implementations of the first assembly 102, such as the components and materials subsequently described with regard to the implementations of FIGS. 3-5.

The pad 105 is configured to be adhesively coupled to a torso 5 of a patient. The pad 105 is formed from a flexible material and is configured to conform to a unique curvature of a region of the torso 5 of the patient. Additionally or alternatively, the contoured pad 105 can include a plurality of segments separated by a flexible material to conform the contoured pad 105 to the curvature of a region of the torso 5 to which it is applied. The pad 105 can be configured to conform with a curvature of a portion of the patient's torso 5, such as lower portion of the torso, an upper anterior portion of the torso, upper posterior portion of the torso, or one or more lateral portions of the torso. In implementations including a pad 105 formed from a conforming material and/or segments, the pad could accommodate various body shapes and sizes and also shape changes associated with movement of the patient's body. For example, the pad 105 could accommodate stretching, expansion, and contraction of a lower region of the torso 5 while a patient stands, walks, sits, or lies prone.

In implementations, the pad 105 can be a sized to accommodate various body sizes. In some implementations, the pad 105 can be manufactured in various sizes accommodating a range of body sizes. The particular shape and size of the pad 105 can be pre-configured or uniquely customized for the patient. For example, various body size measurements and/or contoured mappings may be obtained from the patient, and a uniquely tailored pad 105 may be 3D printed from, for example, any suitable thermoplastic (e.g., ABS plastic). The pad 105 therefore accommodates variable patient sizes and/or contours, and/or some or all portions of the pad can be customized to fit to a patient's particular body size and contour.

In examples, the patient may apply the pad 105 in a uniquely preferred orientation and location. Enabling a patient, in consultation with their caregiver, to place the device 100 in a comfortable location and orientation encourages patient compliance with continuous wear throughout the prescribed duration of wear. For example, the pad 105 can be positioned by a caregiver or physician on the torso 5 of the patient in a first location at the start of the prescribed duration of wear. At least one of the patient, caregiver, and physician may relocate the pad 105 to a second location overlapping with, tangential to, adjacent to, or apart from the first location but within a prescribed region of the torso. For example, the first assembly 102 can be placed initially on a lower anterior region of the torso, along the line of the bottom of a patient's rib cage for comfort and to minimize the appearance of any bulges in clothing worn over the device 100. A patient, caregiver, or physician may remove and re-adhere the pad 105 one inch, for example, in any lateral and/or rotational direction. This provides the patient's skin with an opportunity to breath and regenerate (e.g., slough) and reduces the effects of skin irritation that may be caused by adhesives. By keeping the pad in the region of initial application, the first assembly 102 of the device 100 continues to function in conjunction with the second assembly 107, which is positioned relative to the first assembly and with particular attention to the shock vector traveling between the assemblies 102, 107 and through the heart.

In embodiments, the pad 105 is designed to be durable, flexible, and breathable so as to allow perspiration to evaporate. In embodiments, the pad 105 is non-irritating when contacting skin as described above with regard to skin irritation grading as set forth in Table C.l of Annex C of American National Standard ANSLAAMI/ISO 10993-10:2010, as previously presented. In examples, the pad 105 is generally non-conductive, flexible, water vapor-permeable, and substantially liquid-impermeable or waterproof. The non-conductive flexible, water- vapor permeable pad 105 may comprise or consist of polyurethane, such as TEGADERM polyurethane film (available from 3M), OPSITE polyurethane film (available from Smith & Nephew, London, United Kingdom), or HYDROFILM polyurethane film (available from Hartman USA, Rock Hill, SC). In other examples, the pad 105 can comprise or consist of at least one of neoprene, thermoformed plastic, or injection molded rubber or plastic, such as silicone or other biocompatible synthetic rubber. In examples, the pad 105 is a laminated pad including a waterproof or water resistant layer applied to a relatively more rigid plastic or rubber layer configured to provide structural support for a housing and electronic components disposed therein. In examples the pad 105 is perforated to aid in moisture evaporation from the skin.

In embodiments, the device 100 can include a conductive adhesive layer 138, as indicated in FIG. 3. As described in U.S. Patent No. 9,867,976, titled "LONG-TERM WEAR ELECTRODE," issued on January 16, 2018 (hereinafter the "'976 Patent"), a water-vapor permeable conductive adhesive material can be, for example, the flexible, water vapor-permeable, conductive adhesive material can comprise a material selected from the group consisting of an electro-spun polyurethane adhesive, a polymerized microemulsion pressure sensitive adhesive, an organic conductive polymer, an organic semi-conductive conductive polymer, an organic conductive compound and a semi-conductive conductive compound, and combinations thereof. In an example, a thickness of the flexible, water vapor-permeable, conductive adhesive layer 138 can be between 0.25 and 100 mils. In another example, the water vapor-permeable, conductive adhesive layer 138 can comprise conductive particles. In implementations, the conductive particles may be microscopic or nano-scale particles or fibers of materials, including but not limited to, one or more of carbon black, silver, nickel, graphene, graphite, carbon nanotubes, and/or other conductive biocompatible metals such as aluminum, copper, gold, and/or platinum.

FIG. 3 depicts the first assembly 102, which is a portion of the device 100. The device 100 includes a pad 105 and a housing 120 configured to form a watertight seal with the pad 105. Referring now to FIG. 3, the device 100 includes at least one of a plurality of therapy electrodes 110 integrated with the pad 105. Example therapy electrodes 110 include, for example, conductive metal electrodes, such as those made of stainless steel, tin or aluminum, a conductive ink, or a conductive polymer. The device 100 can also include at least one of a plurality of ECG sensing electrodes 115 integrated with the pad 105. In the implementation of FIG. 3, two ECG sensing electrodes 115a and 115b are shown to be integrated with the pad 105. In examples, the ECG sensing electrodes 115 monitor a patient's ECG information. As described in detail in subsequent examples, the ECG sensing electrodes 115 can be non-polarizable ECG electrodes (e.g., clinical grade Ag/AgCl electrodes) or polarizable electrodes (e.g., electrodes having a metal substrate with an oxide layer, such as a Ta2C>5 coating) configured to measure changes in a patient's electrophysiology to measure the patient's ECG information. Example ECG sensing electrodes 115 include tantalum pentoxide electrodes, as described in, for example, U.S. Patent No. 6,253,099 entitled, "Cardiac Monitoring Electrode Apparatus and Method,". In implementations, the ECG sensing electrodes 115 may be made of a core plastic or metal substrate element that is coated with a thick-film polymeric compound filled with a conductive Ag/Ag/Cl metallic filler.

In some examples, as indicated FIG. 3, at least one therapy electrode 110 and one or more ECG sensing electrodes 115 are formed within the pad 105 such that a skin contact surface of each component is coplanar with or protrudes from the patient contact face of the pad 105. In examples, the therapy electrode 110 and the ECG sensing electrodes 115 are disposed on the patient contact face of the pad 105. In some implementations, the therapy electrode 114 and ECG sensing electrodes 115 are metallic plates (e.g. stainless steel) or substrates that are formed as permanent portions of the device 100. A metallic plate or substrate can be adhered to the pad 105, for example, by a polyurethane adhesive or a polymer dispersion adhesive such as a polyvinyl acetate (PYAc) based adhesive, or other such adhesive. In examples, the plurality of ECG sensing electrodes 115 are a plurality of dry ECG sensing electrodes. In examples, the ECG sensing electrodes 115 are flexible, dry surface electrodes such as, for example, conductive polymer-coated nano-particle loaded polysiloxane electrodes mounted to the pad 105. In some examples, the ECG sensing electrodes 115 are flexible, dry surface electrodes such as, for example silver coated conductive polymer foam soft electrodes mounted to the pad 105. In examples, the ECG sensing electrodes 115 are screen printed onto the pad 105 with a metallic ink, such as a silver-based ink. In implementations, each of the therapy electrodes 110 has a conductive surface adapted for placement adjacent the patient's skin. In some implementations, the therapy electrodes 110 can include a conductive impedance reducing adhesive layer, such as a breathable anisotropic conductive hydrogel disposed between the therapy electrodes and the skin of the patient. In implementations, the at least one ECG sensing electrode 115 can include a breathable hydrogel layer disposed between the at least one ECG sensing electrode 115 and the skin of the patient.

In implementations, the at least one therapy electrode 110 and at least one ECG sensing electrode 115 are manufactured as integral components of the pad 105. For example, the therapy electrode 110 and/or the ECG sensing electrode 115 can be formed of the warp and weft of a fabric forming at least a layer of the pad 105. In implementations, the therapy electrode 110 and/or the ECG sensing electrodes 115 are formed from conductive fibers that are interwoven with non-conductive fibers of the fabric.

The device 100 includes an ECG acquisition and conditioning circuit 125 disposed within the at least one housing 120 and electrically coupled to the plurality of ECG sensing electrodes 115 to provide at least one ECG signal of the patient. In examples, the ECG acquisition and conditioning circuit 125 includes a signal processor configured to amplify, filter, and digitize the cardiac signals prior to transmitting the cardiac signals to a processor 118 of the device 100. The ECG sensing electrodes 115, therefore, can transmit information descriptive of the ECG signals to a sensor interface via the ECG acquisition and conditioning circuit 125 for subsequent analysis.

In examples, as shown in FIG. 3, a therapy delivery circuit 130 is disposed within the at least one housing 120 and configured to deliver one or more therapeutic pulses to the patient through the plurality of therapy electrodes 110 of the device 100. In examples, the processor 118 is disposed within the at least one housing 120 and is coupled to the therapy delivery circuit 130. The processor 118 is configured to analyze the ECG signal of the patient and detect one or more treatable arrhythmias based on the at least one ECG signal. The processor 118 is configured to cause the therapy delivery circuit 130 to deliver at least one defibrillation pulse to the patient on detecting the one or more treatable arrhythmias.

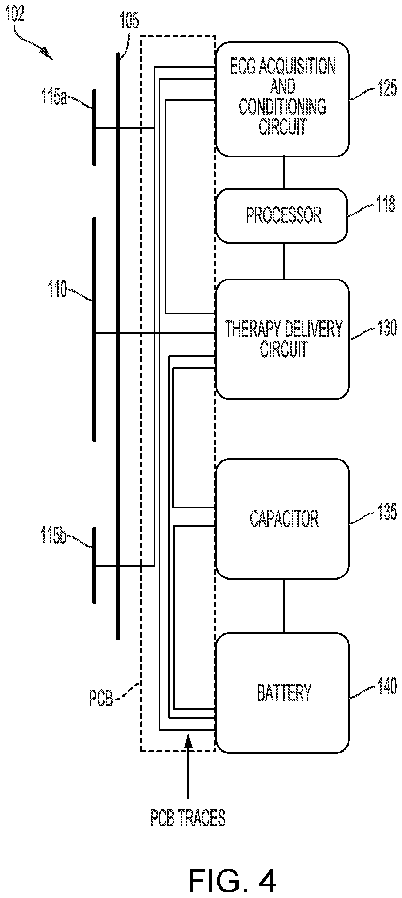

In examples, one or more printed circuit boards 145 connect various circuitry and hardware components (e.g., processor 118, therapy delivery circuit 130, therapy electrodes 110, ECG acquisition and conditioning circuit 125, ECG sensing electrodes 115, etc.) of the first assembly 102. For example, as shown in the schematic of FIG. 4, the printed circuit board 145 can route signals between the therapy delivery circuit 130 and the therapy electrodes 110 and the ECG acquisition and conditioning circuit 125 and the ECG sensing electrodes 115. In implementations of the pad 105 comprising a plurality of segments separated by a flexible material, the one or more circuit boards 145 can be apportioned among some or all of the plurality of segments and, in examples, maybe be electrically interconnected by one or more wires and/or flexible traces or cables.

Continuing with the description of the implementations of the assembly 102 of FIGS. 3-4, in implementations, the therapy delivery circuit 130 is operatively connected to one or more capacitors 135. In implementations the one or more capacitors 135 is a plurality of capacitors (e.g., three, four or more capacitors) that can be switched into a series connection during discharge for a defibrillation pulse. For example, four capacitors of approximately 650 µF can be used. In one implementation, the capacitors can have between 200 to 2500 volt surge rating and can be charged in approximately 5 to 30 seconds from a battery 140 depending on the amount of energy to be delivered to the patient. Additional implementations of capacitor properties and arrangement within the device 100 are provided herein in subsequent sections.

For example, each defibrillation pulse can deliver between 60 to 400 joules (J) of energy. In some implementations, the defibrillating pulse can be a biphasic truncated exponential waveform, whereby the signal can switch between a positive and a negative portion (e.g., charge directions). An amplitude and a width of the two phases of the energy waveform can be automatically adjusted to deliver a predetermined energy amount.

In implementations, the therapy delivery circuit 130 includes, or is operably connected to, circuitry components that are configured to generate and provide the therapeutic shock. As will be described in detail subsequently with regard to implementations of the device 100, the circuitry components include, for example, resistors, one or more capacitors 135, relays and/or switches, an electrical bridge such as an H-bridge (e.g., an H-bridge circuit including a plurality of switches, (e.g. insulated gate bipolar transistors or IGBTs, silicon carbide field effect transistors (SiC FETs), metal-oxide semiconductor field effect transistors (MOSFETS), silicon-controlled rectifiers (SCRs), or other high current switching devices)), voltage and/or current measuring components, and other similar circuitry components arranged and connected such that the circuitry components work in concert with the therapy delivery circuit 130 and under control of one or more processors (e.g., processor 118) to provide, for example, one or more pacing or defibrillation therapeutic pulses.

As introduced previously, at least one housing 120 forms a watertight seal with the pad 105. In examples, such as that of FIG. 3, various circuitry and hardware components (e.g., processor 118, therapy delivery circuit 130, therapy electrodes 110, ECG acquisition and conditioning circuit 125, ECG sensing electrodes 115, PCB 145, etc.) are within a compartment defined by the at least one housing 120 and the pad 105. The housing 120 protects the components thereunder from external environmental impact, for example damage associated with water ingress. Preventing such ingress protects the electronic components of the device 100 from short-circuiting or corrosion of moisture-sensitive electronics, for example, when a patient wears the device while showering. Such features may also protect from other liquid and solid particle ingress.

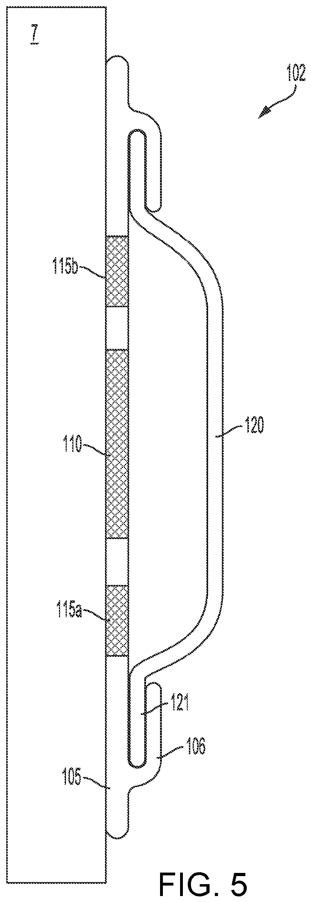

The pad 105 includes one or more receptacles for receiving the at least one housing 120 in a watertight mating. In examples, such as that of FIG. 5, the one or more receptacles comprise a sealing lip 106, and the sealing lip 106 can engage a top surface of the at least one housing 120. In implementations, the sealing lip 106 comprises an elastomeric waterproof material. For example, the one or more receptacles can include a rubber or silicone sealing lip 106 formed integrally with the pad 105 for receiving a flange of the housing in a compression fit seal. For example, the sealing lip 106 and pad 105 can be injection molded as a monolithic structure. In examples, the at least one housing 120 can further include a peripheral flange 121, and the sealing lip 106 securely receives the peripheral flange 121 in a watertight mated configuration. In examples, the housing 120 or one or more of a plurality of housings are removable and/or replaceable. The sealing lip 106 stretches when the housing 120 is pulled away from the pad 105, allowing the housing 120 to be pulled free of the sealing lip 106. Because the sealing lip 106 is elastomeric, the deformation is not permanent, and the sealing lip 106 retracts to a resting state for again receiving the housing 120 and/or plurality of housings in a sealed configuration. In other implementations, housing 120 can be heat welded to the pad 105. In other implementations, the housing 120 can be locked onto the pad 105 and held in compression with a spring loaded clamp. In some or all implementations, the housing and/or pad can included therebetween a deformable waterproof grommet, such as a resilient silicone seal around the perimeter of the interface between the housing and the pad 105.

In addition to forming a watertight seal with the pad, in some examples, the at least one housing 120 is water-resistant and/or coated with a water-resistant coating (e.g., an epoxy coating). Thereby, the device 100 can be worn in the shower without damaging the electrical components disposed within the housing 120. Additionally or alternatively, in implementations at least one of the plurality of ECG sensing electrodes 115, the plurality of therapy electrodes 110, and one or more electrical components of the device (e.g. capacitors 135, therapy delivery circuit 130, processor 118) are housed in one or more water resistant housings 120, or enclosures.

For example, the housing 120 can be constructed to be water-resistant and tested for such in accordance with the IEC 60529 standard for Ingress Protection. For instance, the one or more housings 120 of the device may be configured to have a rating of level 7, protecting against immersion in water, up to one meter for thirty minutes. This enables a patient to wear the device 100 in the bathtub or shower for uninterrupted, continuous use. In implementations, the one or more housings 120 of the device 100 may be multiple coded, including two or more levels. For example, the a housing 120 of the device 100 can maintain a liquid Ingress Protection level of 7, protecting against temporary immersion, and a liquid Ingress Protection level of 5, protecting against water jets.

As described previously, the at least one housing 120 shields one or more of the therapy delivery circuit, the ECG acquisition and conditioning circuit 125, the processor 118, at least one capacitor 135, and at least one power source (e.g., battery 140) from environmental impact. The housing 120 covers and/or surrounds the hardware components therein, protecting them from wear and tear and protecting the patient from contacting high voltage components. The housing 120 protects the components from liquid ingress while the patient is showering, for example.

As described previously with regard to the pad 105, the housing 120 is non-conductive, water vapor-permeable, and substantially liquid-impermeable or waterproof. The housing 120 may comprise or consist of polyurethane, such as TEGADERM polyurethane film (available from 3M), OPSITE polyurethane film (available from Smith & Nephew), or HYDROFILM polyurethane film (available from Hartman USA). In other examples, the pad 105 can comprise or consist of at least one of neoprene, thermoformed plastic, or injection molded rubber or plastic, such as silicone or other biocompatible synthetic rubber. In examples, the housing 120 can include a non-woven laminate material such as at least one spandex, nylon-spandex, and nylon-LYCRA. In examples, the housing 120 can included a thermoformed layer coated with a waterproof or water repellant layer, such as a layer of a non-woven polyurethane fabric material. One or more vapor release valves or through holes can be formed into or disposed through the housing for venting perspiration out of the housing 120. In other examples, the housing 120 can comprise or consist of a fabric having a biocompatible surface treatment rendering the fabric water resistant and/or waterproof. For example, the fabric can be enhanced by dipping in a bath of fluorocarbon, such as Teflon or fluorinated-decyl polyhedral oligomeric silsesquioxane (F-POSS).

In addition to waterproof and/or water resistant characteristics, the adhesively coupled device 100 is volumetrically sized for patient comfort. In examples, the patient-worn monitoring and treatment device 100 has a weight between 250 grams and 2,500 grams. For example, the device 100 can have a weight in a range of at least one of 250 grams and 1,250 grams, 500 grams and 1,000 grams, and 750 grams and 900 grams. Maintaining the weight within such example ranges improves patient comfort. Because the device 100 adheres to the skin of the torso 5 of the patient, examples of the device 100 include weight distribution and adhesive features for encouraging patient compliance by improving comfort and sustaining attachment throughout the prescribed duration.

Referring to FIGS. 2A, 2B, and 3, a vertical axis 137 of the first and second assemblies 102, 107 is antiparallel to the force of gravity when the patient is standing or sitting and when the adhesively coupled assemblies are affixed to the patient per the medical instructions for application and use.

There are a number of forces exerted on an adhesive joint that may cause it to fail prematurely. These include one or more of the following forces:
1) Tensile force is pull exerted equally over the entire joint. With tensile force on an adhesive bond, the pull direction is normal to the adhesive bond;
2) Shear force on an adhesive bond is pull directed across the adhesive, parallel to the adhesive bond, forcing the substrates to slide over each other;
3) Cleavage force is pull concentrated at one edge of the adhesive joint, exerting a prying force on the bond. The other edge of the joint is theoretically under zero stress; and/or
4) Peel force is concentrated along a thin line at the edge of the bond where one substrate is flexible. The line is the exact point where an adhesive would separate if the flexible surface were peeled away from its mating surface. Once peeling has begun, the stress line remains in front of the advancing bond separation.

The leverage effect of cleavage and peel forces concentrate stress at smaller areas of the bond causing failure at lower force levels than those observed in tension and shear. By exploiting the relative component density inhomogeneities such as by placement of the components within the housing, and distributing the electronic components such that the center of mass or center of gravity 147 for the housing 120 is below the volumetric center 150 of the housing 120 in their positions along the vertical axis 137, the delamination forces, such as the cleavage and peel, can be minimized. This assists with securing the first assembly 102 to the patient while minimizing any undesired partial or full separation of the device 100 from the skin of the patient during the prescribed duration of wear. If the pad 105 pulls away from the skin of the patient, the therapy electrodes 110 and ECG sensing electrodes 115 can lose contact with the skin, preventing proper monitoring of the patient.

In implementations, as shown in FIG. 3, the center of mass or center of gravity 147 for the first assembly 102 is below the volumetric center 150 of the housing 120 relative to the vertical axis 137 such that the ratio of the distance V1 between the center of mass or center of gravity 147 and the inferior margin line 136 of the housing 120 divided by the distance V2 between the volumetric center 150 and the inferior margin line 136 is less than 90%. As shown in the example of FIG. 3, the inferior margin line 136 is the line (or plane) tangential to a bottom end 123 of the housing 120. In other implementations, the ratio of the distances V1/V2 may be less than 80%. In other implementations, the ratio of the distances V1/V2 can be less than 75%. In other implementations, the ratio of the distances V1/V2 can be less than 70%. In other implementations, the ratio of the distances V1/V2 can be less than 50%. In other implementations, the ratio of the distances V1/V2 can be less than 30%. In other implementations, the ratio of the distances V1/V2 can be less than 20%. In implementations, the ratio of the distances V1/V2 can be in a range of between 1% to 90%. In implementations, the ratio of the distances V1/V2 can be in a range of between 5% to 80%. In implementations, the ratio of the distances V1/V2 can be in a range of between 10% to 70%. In some implementations, there may be more than one vertical axis 137 defined, e.g. a vertical axis oriented based on the patient awake and standing, and a second vertical axis based on the patient asleep on their backs. In order to meet the above criteria for the ratio of distances for both orientations, the center of gravity of center of mass 147 is located in the lower, rear quadrant of the housing 120. In examples, the heaviest electrical components (e.g., the at least one capacitor 135 and battery 140) are disposed below the volumetric center 150 of the first assembly 102.

As described previously with regard to the examples of FIGS. 2A and B, the device 100 includes a first assembly 102 and a separate second assembly 107 coupled to the first assembly 102 and configured to be adhesively coupled to the torso 5 of the patient. The second assembly 107 can be coupled to the first assembly 102 with one or more conductive threads or wires 116 and configured for delivering current for an electrical pulse, such as a, for example, a 360J defibrillation pulse.

The second assembly 107 can include breathable and non-irritating materials and adhesives as described above with regard to the first assembly 102 and the pad 105. In examples, the second assembly 107 includes a second one of the plurality of therapy electrodes 110 integrated with a pad 109 of the second assembly 107 and in wired communication with the therapy delivery circuit 130. In examples, the second assembly 107 includes an ECG sensing electrode 115. Because the first assembly 102 contains the therapy delivery circuit, and various other electrical components, the second assembly 107 can be more compact than the first assembly. For example, the second assembly can be a low profile adhesive pad including a therapy electrode 110 and an ECG electrode 115 and having a pad thickness of about 0.1cm to 2 cm. In one example, the pad 109 of the second assembly 107 can have a width W2 of between 1 cm and 4 cm and a length L2 of between 2 cm and 10 cm. In one example, the pad 109 circumscribes an area (e.g., an "area footprint") of the patient's skin of approximately 100 square centimeters (e.g., 0.01 square meters). Components thereon and/or therein have a cumulative weight ranging from 0.05 kg to 1.0 kg

In embodiments, the pad 109 of the second assembly is designed to be durable, flexible, and breathable so as to allow perspiration to evaporate. In embodiments, the pad 109 is non-irritating when contacting skin as described above with regard to skin irritation grading as set forth in Table C.l of Annex C of American National Standard ANSEAAMEISO 10993-10:2010, as previously presented. In examples, the pad 109 is generally non-conductive, flexible, water vapor-permeable, and substantially liquid- impermeable or waterproof. The non-conductive flexible, water- vapor permeable pad 105 may comprise or consist of polyurethane, such as TEGADERM polyurethane film (available from 3M), OPSITE polyurethane film (available from Smith & Nephew), or HYDROFILM polyurethane film (available from Hartman USA). In other examples, the pad 109 can comprise or consist of at least one of neoprene, thermoformed plastic, or injection molded rubber or plastic, such as silicone or other biocompatible synthetic rubber. In examples, the pad 109 is a laminated pad including a waterproof or water resistant layer applied to a relatively more rigid plastic or rubber layer configured to provide structural support for a housing and electronic components disposed therein. In examples the pad 109 is perforated to aid in moisture evaporation from the skin.

In embodiments, the second assembly 107 can include a conductive adhesive layer. As described in the '976 Patent, a water-vapor permeable conductive adhesive material can be, for example, a material selected from the group consisting of poly(3 ,4-ethylene dioxitiophene), doped with poly( styrene sulfonate), (PEDOT:PSS) poly(aniline) (PANI), poly(thiopene)s, and poly(9,9- dioctylfluorene co-bithiophen) (F8T2), and combinations thereof. Such polymers can be printed as a flexible, water vapor-permeable, conductive adhesive layer using such methods as inkjet printing, screen printing, offset printing, flexo printing, and gravure printing. In an example, a thickness of the flexible, water vapor-permeable, conductive adhesive material can be between 0.25 and 50 mils. In another example, the water vapor-permeable, conductive adhesive material can comprise conductive particles. In implementations, the conductive particles may be microscopic or nano-scale particles or fibers of materials, including but not limited to, one or more of carbon black, silver, nickel, graphene, graphite, carbon nanotubes, and/or other conductive biocompatible metals such as aluminum, copper, gold, and/or platinum.

In examples, as shown in FIGS. 2A-B, the second assembly 107 includes at least one of a plurality of therapy electrodes 110 integrated with the pad 109. Example therapy electrodes 110 include, for example, conductive metal electrodes, such as those made of stainless steel, tin or aluminum, a conductive ink, or a conductive polymer. In examples, the second assembly can also include at least one of a plurality of ECG sensing electrodes 115 integrated with the pad 109. As described with regard to the first assembly 102, the ECG sensing electrodes 115 of the second assembly 107 can be non-polarizable ECG electrodes (e.g., clinical grade Ag/AgCl electrodes) or polarizable electrodes (e.g., electrodes having a metal substrate with an oxide layer, such as a Ta20s coating) configured to measure changes in a patient's electrophysiology to measure the patient's ECG information. Example ECG sensing electrodes 115 include tantalum pentoxide electrodes, as described in, for example, U.S. Patent No. 6,253,099 entitled "Cardiac Monitoring Electrode Apparatus and Method,". In implementations, the ECG sensing electrodes 115 may be made of a core plastic or metal substrate element that is coated with a thick-film polymeric compound filled with a conductive Ag Ag/Cl metallic filler.

In some examples, at least one therapy electrode 110 and one or more ECG sensing electrodes 115 are formed within the pad 109 such that a skin contact surface of each component is coplanar with or protrudes from the patient contact face of the pad 105. In examples, the therapy electrode 110 and the ECG sensing electrodes 115 are disposed on the patient contact face of the pad 105. In some implementations, the therapy electrode 114 and ECG sensing electrodes 115 are metallic plates (e.g. stainless steel) or substrates that are formed as permanent portions of the second assembly 107. A metallic plate or substrate can be adhered to the pad 109, for example, by a polyurethane adhesive or a polymer dispersion adhesive such as a polyvinyl acetate (PVAc) based adhesive, or other such adhesive. In examples, the ECG sensing electrodes 115 are flexible, dry surface electrodes such as, for example, conductive polymer-coated nano-particle loaded polysiloxane electrodes mounted to the pad 109. In some examples, the ECG sensing electrodes 115 are flexible, dry surface electrodes such as, for example silver coated conductive polymer foam soft electrodes mounted to the pad 109. In examples, the ECG sensing electrodes 115 are screen printed onto the pad 109 with a metallic ink, such as a silver-based ink. In implementations, each of the therapy electrodes 110 has a conductive surface adapted for placement adjacent the patient's skin. In some implementations, the therapy electrodes 110 can include a conductive impedance reducing adhesive layer, such as a breathable anisotropic conductive hydrogel disposed between the therapy electrodes and the skin of the patient. In implementations, the at least one ECG sensing electrode 115 can include a breathable hydrogel layer disposed between the at least one ECG sensing electrode 115 and the skin of the patient.

In implementations, the at least one therapy electrode 110 and at least one ECG sensing electrode 115 are manufactured as integral components of the pad 109. For example, the therapy electrode 110 and/or the ECG sensing electrode 115 can be formed of the warp and weft of a fabric forming at least a layer of the pad 109. In implementations, at least one of the therapy electrode 110 and the ECG sensing electrodes 115 is formed from conductive fibers that are interwoven with non-conductive fibers of the fabric.

In examples, the device 100 can include a third assembly configured to be adhesively coupled to the torso of a patient. The third pad can include one of the plurality of therapy electrodes 110 integrated with a pad of the third assembly and in wired communication with the therapy delivery circuit 130. The third assembly can have similar characteristics as those described above with regard to the second assembly 107. In examples, the third pad can be configured for adhesively attaching to a posterior portion of the torso 5 of the patient, between the shoulder blades of the patient, for example, while the first assembly 102 is positioned along the lower ridge of the rib cage and the second assembly 107 is positioned on an upper anterior portion of the torso 5 adjacent to the apex of the heart. In examples, the third assembly includes a third pad configured to be adhesively coupled to the torso of the patient adjacent the atria.

Implementations of a patient-worn arrhythmia monitoring and treatment device 100 can include at least two pads (e.g., pads 105 and 109) including an adhesive layer (e.g., adhesively layer 138) configured to affix each one of the at least two pads to skin on the torso 5 of the patient and one or more removable wearable supports and/or support garments for offsetting one or more forces such as peel forces, shear forces, cleavage forces, and tensile forces and retaining the at least two adhesively affixed pads in contact with the torso of the patient. In such implementations, the removable wearable supports and/or support garments assist with preventing the device from pulling on the skin of the patient and therefore increase and/or ensure patient comfort throughout the duration of wear, especially during activities involving patient movement and/or positioning that would impart forces on the adhesive layer and pulling of the patient's skin by the at least two adhesively affixed pads. Ensuring patient comfort removes an impediment to patient compliance with wearing the device throughout the prescribed durations. Such removable wearable supports and/or garments can be especially beneficial for selective wear during certain activities of finite duration including at least one of athletic activities, showering, bathing, and sleeping.

In embodiments, such as those of FIGS. 6A and C, the first and second assembly patient-worn arrhythmia monitoring and treatment device 100B and E, can both be placed on an anterior portion of the torso 5. The second assembly 107 of the device 100 can be placed on the torso 5 above the patient's right nipple, and the first assembly 102 is placed on the left lateral side of the patient's torso 5 opposite placement of the second assembly 107. In other implementations, such as those of FIGS. 6B and D, the second assembly 107 can be placed on a posterior, upper right portion of the torso and the first assembly 102 is placed on the left lateral side of the patient's torso 5 opposite placement of the second assembly 107. As shown in FIGS. 6C-D, in embodiments the device 100D-E includes a first assembly 102 and second assembly 107 in wired connection. Alternatively, as shown in FIGS. 6A-B, in embodiments the device 100B-C includes a first assembly 102 and a second assembly 107 in wireless communication, for example when the device is used for monitoring for a cardiac condition. In some examples, the wire 116 can be detachable, and the device can prompt the patient to attach the wire 116 to the first and second assemblies 102, 107 when the device detects a cardiac condition requiring treatment.

In implementations, as described with regard to the embodiments of FIGS. 2A-3, the device 100B-E of FIGS. 6A-D includes at least two pads 105, 109 including an adhesive layer 138 configured to affix each one of the at least two pads 105, 109 to skin on the torso 5 of the patient. At least one of a pair of sensing electrodes 115 are disposed on each one of the at least two pads. The pair of sensing electrodes 115 are configured to sense surface ECG activity of the patient. At least one of a pair of therapy electrodes are disposed on each one of the at least two pads. The pair of therapy electrodes are configured to deliver one or more therapeutic pulses to the patient. A controller 20, as described with regard to the embodiments of FIGS. 2A-3, is coupled to one of the at least two pads and is in communication with the pairs of sensing and therapy electrodes. The controller 20 is configured to monitor for cardiac arrhythmias based on the sensed surface ECG activity of the patient and cause the delivery of one or more therapeutic pulses to the patient. In implementations, the controller is disposed within a housing that is coupled to the pad 105 of the first assembly 102. The controller 20 includes, one or more of capacitors, batteries, processors, printed circuit boards, ECG acquisition and conditioning circuitry, and high-voltage therapy control circuitry disposed within the housing, and the first assembly 102 can weight around 500 grams - 10kgs.

In implementations, the patient- worn arrhythmia monitoring and treatment device includes a removable garment. In implementations, such as those of FIGS. 6A-D, the removable garment is a garment configured to hold the at least two pads in compression against the torso so as to minimize or eliminate delamination of the adhesive from the skin. By maintaining the adhesive coupling between the skin and the adhesive intact, sensor signal noise and other artifacts additionally can be minimized or substantially eliminated. In implementations, the removable garment is configured to be worn over at least one of the at least two torso-mounted pads and apply a compression force to maintain contact of the at least one of the at least two torso-mounted pads with the torso. In implementations, the removable garment 51, 53 includes a compression portion. The compression portion is configured to immobilize the garment 51, 53 relative to a skin surface of the thoracic region 105 of the patient by exerting one or more compression forces against the thoracic region. In implementations, the garment 51, 53 is configured to exert the one or more compression forces in a range from 1.72 to 51.71 mbar (0.025 to 0.75 psi) to the thoracic region 105. For example, the one or more compression forces can be in a range from 3.45 mbar to 48.26 mbar (0.05 psi to 0.70 psi), 5.17 mbar to 46.54 mbar (0.075 psi to 0.675 psi), or 6.89 to 44.82 mbar (0.1 to 0.65 psi)

In an example, the removable garment 51, 53 exerts compression forces against the skin of the patient by one or more of manufacturing all or a portion of the garment 51, 53 from a stretchable fabric, providing one or more tensioning mechanisms in and/or on the garment, and providing a cinching closure mechanism 52 for securing and compressing the garment 51, 53 about the torso 5. Alternatively, or in addition to the stretchable fabric, a compressible pillow can be located on the inner surface of the removable garment 51, 53 in the area positioned just over the at least one of the at least two torso-mounted pads. The compressible pillow is configured to provide localized compressive forces to the region in the vicinity of the at least one of the at least two torso-mounted pads. In implementations, the compressible pillow can be, for example, a fluid-filled bladder such as a gas-filled bladder, or a piece of foam. The compressible pillow can have a thickness, for example, of between about 6.35 - 25.4 mm (0.25 - 1.0 inch)

In implementations, the garment can be a shoulder strap, a vest, a shirt, a belt, a harness, a bandeau, and a sash, or a combination of one or more of the foregoing. For example, as shown in the implementations of FIGS. 6A and 6C, the garment can be a belt 51, or waistband, configured for supporting the first assembly 102 and relatively heavier components included therein (e.g., the first assembly 102 weighing around 500 grams-10 kgs). In implementations, the belt 51 can include a tensioner 52 for tightening and/or loosening the belt 51 about the torso 5 of the patient. In implementations, the tensioner 52 can also fasten the belt 51 about the torso 5 of the patient, such a hook and loop fastener system or a ratchet strap and buckle assembly.

In embodiments, the first assembly 102 can be configured to integrate with the wearable support. For example, the first assembly 102 can form a linking portion of a belt removable affixed to the housing 120 with fasteners or removable threaded through receiving loops to tension the first assembly 102 against the torso so that the sensors integrated with the pad 105 are immobilized in contact the skin of the patient. In some embodiments, such as those of FIGS. 6A-D, the first assembly 102 and/or second assembly 107 can be covered by the wearable support to enhance adhesion of the adhesive layer 138 and hold the first pad 105 and/or second pad 109 in place against the skin. For example, as indicated by the broken lines in FIGS. 6A-D, the removable garment can be a belt 51 positioned on a lower torso region or a sash 53 extending diagonally across the torso 5 and covering the first assembly 102 and/or second assembly 107. In embodiments, the belt 51 and sash 53 can include a closure mechanism 52. In implementations, the removable garment 51, 53 comprises a closure mechanism including at least one of a ratchet strap, an adjustable buckle, an extendable and moveable hook and loop fastener strip, a tie, a snap, and a button. The closure mechanism can be a tensioner configured for tightening the wearable support about the torso 5, adding compression force to the first assembly 102 and/or second assembly 107 and assisting with maintaining contact of the first and second assemblies 102, 107 with the torso 5.

As described previously, in some examples, the second assembly 107 can include only relatively lighter components such as one or more therapy electrodes 110 and/or one or more ECG sensing electrodes 115. In alternative implementations, the second assembly 107 can include one or more of the heavier components (e.g., the second assembly weighing around 500 grams-10 kgs), such as one or more capacitors, batteries, and/or the therapeutic circuitry when compared to the first assembly 102. Providing additional wearable support, such as a sash 53 as shown in FIGS. 6B and 6D, for example, can assist with retaining the relatively heavier second assembly 107 that includes heavier components, against an upper region of the torso 5. Providing such additional wearable support assists with preventing the second assembly 107 from pulling uncomfortably on the skin and/or peeling away from the skin of the patient while adhesively attached.

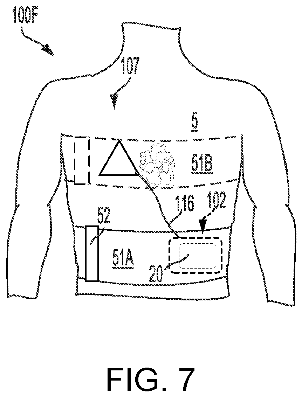

In implementations such as that of FIG. 7, the device 100F, can include two removable garments configured to be worn about the torso 5 of the patient, a first removable garment 51A configured to be worn over the first assembly 102 and a second removable garment 51B configured to be worn over the second assembly 107. In implementations, a controller 20 is disposed within a housing that is coupled to the first pad 105 of the first assembly 102. The controller includes, one or more of capacitors, batteries, processors, printed circuit boards, ECG acquisition and conditioning circuitry, and high-voltage therapy control circuitry disposed within the housing. The second assembly 107 includes a second pad 109 and a therapy electrode 110 and ECG sensing electrode 115. Because the second assembly 107 is lighter relative to the first assembly 102 and less likely to pull away from the skin of the torso 5, in implementations, the second removable garment 51B can impart a smaller compression force than the first removable garment 51A.

As described previously with regard to implementations of the patient- worn arrhythmia monitoring and treatment device, a first of the at least two pads has a first weight and a second of the at least two pads has a second weight, wherein the first weight is greater than the second weight. In implementations, the first of the at least two pads weighs between 1kg-2.5kg and the second of the at least two pads weighs between 0.5kg-1kg, and the first of the at least two pads is the one of the at least two pads to which the controller is coupled. In implementations, as described previously for example with regard to FIGS. 3 and 6A-D, the controller 20 is disposed within a housing that is coupled to one of the at least two pads. The controller 20 can comprise one or more of capacitors, batteries, processors, printed circuit boards, ECG acquisition and conditioning circuitry, and high-voltage therapy control circuitry disposed within the housing.

As described previously, the removable garment is configured for selective wear during certain activities of finite duration including at least one of athletic activities, showering, bathing, and sleeping such that the one of the at least two pads to which the controller is coupled is immobilized on the torso 5. As described previously, in implementations, the removable garment can be a compression garment configured to apply pressure to the adhesive layer of at least one of the at least two pads to maintain contact of the sensors integrated therein with the skin of the torso of the patient. This stabilizing force reduces motion related noise artifacts in the sensor signals and improves patient comfort by minimizing forces associated with adhesive tearing or peeling away from the patient's skin. In implementations, in addition to or alternatively to immobilizing one or both of the at least two pads by an application of compression force, the removable garment can immobilize the one of the at least two pads to which the controller is coupled by at least partially supporting a weight of the one of the at least two pads to which the controller is coupled. In implementations, the removable garment can include a retention feature configured to facilitate the selective wear during certain activities by allowing separation of the removable garment from the one of the at least two pads to which the controller is coupled.

In implementations, the retention feature can include a fastener including at least one of hook and loop fastener strip, a tie, a snap, a button, or a combination of one or more of the foregoing. For example, the fastener can engage with a corresponding mating portion disposed on or integrated with the housing 120 that is coupled to one of the at least two pads. In implementations, the retention feature includes an interlock disposed on an interior surface of the removable garment. The interlock can be configured to engage a corresponding mating portion disposed on an exterior surface of the housing. For example, the interlock can be engaged by an application of hand force pressing the interlock on a mating portion and can disengage by an application of hand force pulling the interlock free of the engagement portion. The disengagement hand force can be less than the peel strength of the adhesive such that removal of the removable garment does not peel the adhesive layer of the one of the at least two pads from the torso of the patient.

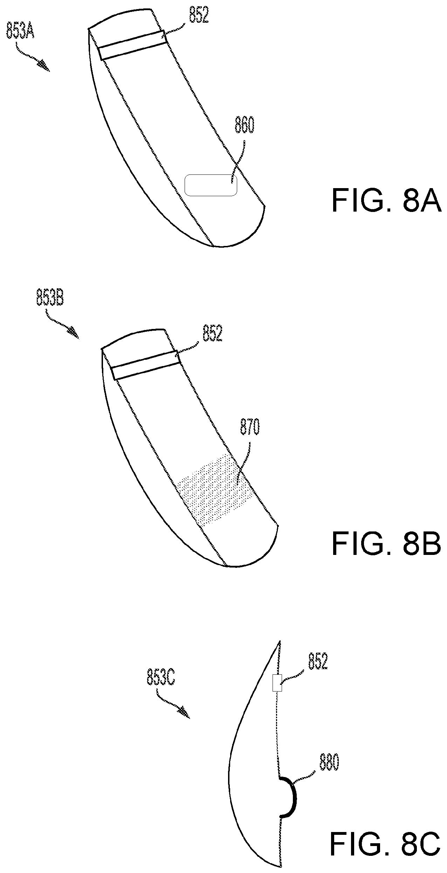

In implementations, such as that of FIG. 8A, a removable garment 853A can be a cross body sash that includes a hole 860 therein that is sized and shaped to accommodate the housing 120 of the first assembly 107 when inserted through the hole 860. The hole 860 can be an opening in the fabric of the removable garment 853A. In implementations, the opening can be slightly smaller than the largest planar cross-sectional surface area of the housing, similar to a button hole configuration, such that housing can protrude from the opening with the pad 105 securely compressed beneath the removable garment 853A. In implementations, the hole 860 can include an elasticized mouth such that the opening expands to accommodate the housing 120 upon insertion and then contracts about the base of the housing 120 to form a secure engagement.

In implementations, such as that of FIG. 8B, a removable garment 853B can be a cross body sash that includes a non-slip surface 870. The non-slip surface 870 is a retention feature disposed on an interior surface of the removable garment 853B such that the removable garment remains stationary relative to the housing 120. The non-slip surface 870 can be, for example, one or more high friction fabrics or materials integrated with the fabric of the removable garment 853. For example, the non-slip surface 870 can be a rough, textured fabric having a non-smooth surface topography. In implementations, the non-slip surface 870 can be a patch of a silicone or a series of silicone treads covering a portion or all of the interior surface of the removable garment.

In implementations, such as that of FIG. 8C, a removable garment 853C can be a cross body sash that includes a conforming cup 880. The conforming cup 880 can be formed as non-removable portion of the removable garment such that the removable garment remains stationary relative to the housing when the housing 120 is received in the conforming cup. In implementations, the conforming cup 880 can be formed from a molded plastic or rubber material configured to engage the contours of the housing 120 in a fitted overlap. In implementations, the conforming cup 880 can be flexible and/or compliant so as to stretch over the housing, firmly compressing the housing 120 against the torso 5.

In implementations, the removable garment 51, 53, 853A-C can be an unbroken loop and can include a compression portion that comprises a stretchable fabric. The removable garment 51, 53, 853A-C can be configured to stretch over the shoulders or hips of the patient and contract when positioned about the torso. The removable garment 51, 53, 853A-C can comprise or consist of an elastic polyurethane fiber that provides stretch and recovery. For example, the removable garment 51, 53, 853A-C can comprise or consist of at least one of neoprene, spandex, nylon-spandex, nylon-LYCRA, ROICA, LINEL, INVIYA, ELASPAN, ACEPORA, and ESPA.

During an initial fitting, the physician, caregiver, or a patient service representative (PSR) can select a removable garment 51, 53, 853A-C sized to fit the patient. For example, the physician, caregiver, or PSR can measure a circumference or cross body dimension of the torso 5 and select a removable garment 51, 53, 853A-C having a circumference within about 75% to about 95% of the encircling measurement of the torso 5. During the initial fitting, the removable garment 51, 53, 853A-C can be fitted by the physician, caregiver, or PSR such that one or more retention features of the garment are positioned on the torso 5 to assist with positioning and/or applying an adhesive pad 105 to the torso 5. For example, the removable garment 853A-C of FIGS. 8A-C can be used to align the retention feature 860, 870, 880 with a preferred position of the pad 105 on the torso prior to the application of the adhesive layer. With the pad 105 aligned with the retention feature 860, 870, 880, the adhesively layer 138 can be exposed to the skin of torso, for example by removal of a release layer, and compressed against the skin the by removable garment 853A-C. Once the adhesive has set so that the pad is affixed to the torso, the removable garment 853A-C can be removed for subsequent temporary uses by the patient.

In some implementations, the compression portion comprises an elasticized thread disposed in the removable garment 51, 53, 853A-C. The compression portion can comprise an elasticized panel disposed in the removable garment 51, 53, 853A-C, the elasticized panel comprising a portion of the removable garment 51, 53, 853A-C spanning less than the entirety of the removable garment 51, 53, 853A-C. For example, the removable garment 51, 53, 853A-C can include one or more mechanically joined sections forming a continuous length or unbroken loop. The one of the one or more sections can comprise a stretchable fabric and/or elasticized thread interspersed with non-stretchable or relatively less stretchable portions. In other embodiments, the removable garment 51, 53, 853A-C can include a compression portion comprising an adjustable tension element, such as one or more cables disposed in the removable garment 51, 53, 853A-C and configured to be pulled taut, e.g., held in tension by one or more pull stops. In some embodiments, the removable garment 51, 53, 853A-C can include one or more visible or mechanical tension indicators configured to provide a notification of the removable garment 51, 53, 853A-C exerting compression forces against the torso 5 in a range from about 1.72 mbar to 51.71 mbar (0.025 psi to 0.75 psi)

Additionally or alternatively, the removable garment 51, 53, 853A-C can have proportions and dimensions derived from patient-specific thoracic 3D scan dimensions. From a 3-dimensional scan of the torso 5 of the patient, a removable garment 51, 53, 853A-C can be sized to fit proportions, dimensions, and shape of the torso 5. In implementations, for example, various body size measurements and/or contoured mappings may be obtained from the patient, and one or more portions of the removable garment 51, 53, 853A-C can be formed of a plastic or polymer to have contours accommodating one or more portions of the torso 5 in a nested fit. For example one or more portions of the removable garment 51, 53, 853A-C may be 3D printed from, for example, any suitable thermoplastic (e.g., ABS plastic) or any elastomeric and/or flexible 3D printable material. For example the removable garment 51, 53, 853A-C may include at least one curved rigid or semi-rigid portion for engaging the patient's shoulder and/or lateral sides, under the arms. The at least two curved portions add rigid structure that assists with preventing the removable garment 51, 53, 853 A-C from shifting or rotating about the thoracic region and therefore prevents the band from pulling the adhesive layer of the first and/or second pad 105, 109 away from the skin of the torso 5.

In implementations, the removable garment 51, 53, 853A-C comprises a breathable, skin- facing layer including at least one of a compression padding, a silicone tread, and one or more textured surface contours. The breathable material and compression padding enable patient comfort throughout the duration of wear and the silicon tread and/or one or more surface contours assist with immobilizing the removable garment 51, 53, 853A-C relative to the skin surface of the torso 5.

Implementations of the device 100 in accordance with the present disclosure may exhibit a moisture vapor transmission rate (MVTR) of, for example, between about 600 g/m2/day and about 1,400 g/m2/day when worn by a subject in an environment at room temperature (e.g., about 25° C) and at a relative humidity of, for example, about 70%. In implementations, the device 100 has a water vapor permeability of 100g/m2/24 hours, as measured by such vapor transmission standards of ASTM E-96-80 (Version E96/E96M-13), using either the "in contact with water vapor" ("dry") or "in contact with liquid" ("wet") methods. Such test methods are described in in U.S. Patent No. 9,867,976, titled "LONG TERM WEAR ELECTRODE," issued on January 16, 2018 (hereinafter the "'976 Patent"). In implementations, the removable garment 51, 53, 853A-C comprises one or more moisture wicking fabrics for assisting with moving moisture away from the skin of the torso 5 and improving patient comfort during wear. For example, the removable garment 51, 53, 853A-C can be selectively worn during periods of exercise, when the patient is perspiring. By moving moisture away from the skin, the removable garment 51, 53, 853A-C encourages patient compliance with wearing the additional support to counteract forces that otherwise degrade the adhesive attachment of the at least two pads 105, 109 to the torso 5.

In implementations, the removable garment 51, 53, 853A-C, can be water vapor-permeable, and substantially liquid-impermeable or waterproof. In implementations, a portion of the removable garment 51, 53, 853 A-C comprises a water resistant and/or waterproof fabric covering at least one of the first and second pads 105, 109, and the wire 116, and a portion of the band comprises a water permeable, breathable fabric having a relatively higher moisture vapor transmission rate than the water resistant and/or waterproof portions. In examples, the removable garment 51, 53, 853 A-C can comprise or consist of a fabric having a biocompatible surface treatment rendering the fabric water resistant and/or waterproof. For example, the fabric can be enhanced by dipping in a bath of fluorocarbon, such as Teflon or fluorinated-decyl polyhedral oligomeric silsesquioxane (F-POSS). Additionally or alternatively, the removable garment 51, 53, 853A-C can comprise or consist of a fabric including anti-bacterial and/or anti-microbial yarns. For example, these yarns can include a base material of at least one of nylon, polytetrafluoroethylene, and polyester. These yarns can be for example, one or more of an antibacterial silver coated yarn, antibacterial DRAYLON yarn, DRYTEX ANTIBACTERIAL yarn, NILIT BREEZE and NILIT BODYFRESH. In implementations, an outer surface of the removable garment 51, 53, 853A-C can comprise one or more patches of an electrostatically dissipative material such as a conductor-filled or conductive plastic in order to prevent static cling of a patient's clothing. Alternatively, in embodiments, the removable garment 51, 53, 853A-C comprises a static dissipative coating such as LICRON CRYSTAL ESD Safe Coating (TECHSPRAY, Kennesaw, GA), a clear electrostatic dissipative urethane coating.

Additionally or alternatively, the removable garment 51, 53, 853A-C includes low skin-irritation fabrics and/or adhesives. In embodiments, the removable garment 51, 53, 853A-C may be worn continuously by a patient for a long-term duration (e.g., duration of at least one week, at least 30 days, at least one month, at least two months, at least three months, at least six months, and at least one year) without the patient experiencing significant skin irritation. For example, a measure of skin irritation can be based on skin irritation grading of one or more as described previously with regard to Table 1.

Additionally or alternatively, in implementations, the removable garment 51, 53, 853A-C can include an adhesive configured to immobilize the removable garment 51, 53, 853A-C relative to the torso 5 of the patient. In implementations, the adhesive is configured to be a removable and/or replaceable adhesive patch for preventing the removable garment 51, 53, 853A-C from shifting, rotating, or slipping relative to the skin of the thoracic region. In implementations, the adhesive has a low skin irritation grading (e.g., a grading of 1) in accordance with the method set forth in American National Standard ANSI/AAMI/ISO 10993-10:2010, previously described. In implementations, once the patient is wearing the removable garment 51, 53, 853A-C, the patient can insert one or more adhesive patches between the removable garment 51, 53, 853A-C and the skin. In implementations, the patient can swap out one or more adhesive patches with one or more new adhesive patches in the same or a different location between the removable garment 51, 53, 853A-C and the skin of the torso 5. For example, the patient may swap out the one or more adhesive patches on a daily schedule or may use the adhesive patches selectively during periods of high activity, such as while exercising. The adhesives can include biocompatible adhesives, such as pressure-sensitive adhesives having tack, adhesion, and cohesion properties suitable for use with a medical device applied to skin for short term and long-term durations. These pressure sensitive adhesives can include polymers such as acrylics, rubbers, silicones, and polyurethanes having a high initial tack for adhering to skin. These pressure sensitive adhesives also maintain adhesion during showering or while a patient is perspiring. The adhesives also enable removal without leaving behind uncomfortable residue. For example, such an adhesive can be a rubber blended with a tackifier.

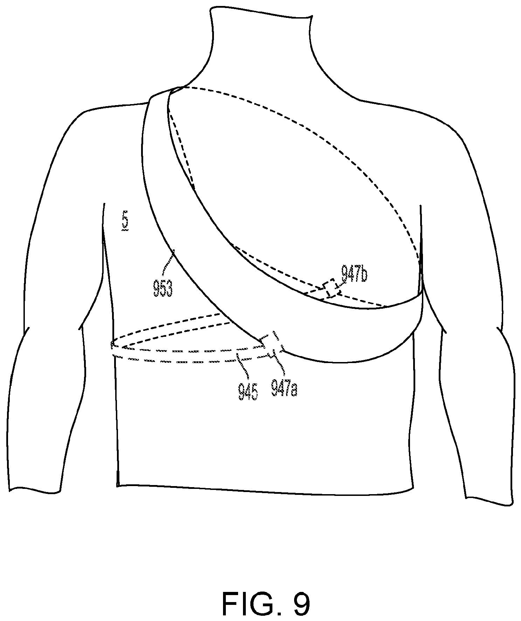

In implementations, such as that of FIG. 9, in addition to or alternative to an adhesive, the removable garment 953 can include an auxiliary strap 945, shown in dashed line in FIG. 9 to indicate optional use. In implementations, a patient optionally may attach the auxiliary strap 945 around the torso 5. In implementations, the auxiliary strap 945 can attach to an anterior portion of the removable garment 953 with a connector 947a such as a hook and look fastener, a clip, buttons, or snaps. Similarly, the auxiliary strap 945 can attach to a posterior portion of the removable garment 953 with a connector 947b such as a hook and look fastener, a clip, buttons, or snaps. The optionally worn auxiliary strap 945 is configured to prevent the removable garment 953 from shifting and/or rotating. A patient may attach the auxiliary strap 945 during periods of high activity, such as during exercise.

As described above, the teachings of the present disclosure can be generally applied to external medical monitoring and/or treatment devices (e.g., devices that are not completely implanted within the patient's body). External medical devices can include, for example, ambulatory medical devices that are capable of and designed for moving with the patient as the patient goes about his or her daily routine. An example ambulatory medical device can be a wearable medical device such as a wearable cardioverter defibrillator (WCD), a wearable cardiac monitoring device, an in-hospital device such as an in-hospital wearable defibrillator, a short-term wearable cardiac monitoring and/or therapeutic device, and other similar wearable medical devices.

As describe herein in implementations, an adhesively coupled patient-worn arrhythmia monitoring and treatment device can include additional, removable wearable supports and/or support garments for offsetting one or more forces such as peel forces, shear forces, cleavage forces, and tensile forces and retaining the wearable device in contact with the torso of the patient. In such implementations, the removable wearable garment assists with preventing the adhesively coupled patient-worn arrhythmia monitoring and treatment device from pulling on the skin of the patient. This therefore increases and/or ensures patient comfort during activities and/or assuming certain body positions that otherwise impart forces on the skin of the patient. Such activities or positions can include exercising, bathing, or sleeping, when the patient's body is in motion or stretching and moving in ways that would disturb the adhesively coupled wearable device. Ensuring patient comfort removes an impediment to patient compliance with wearing the device throughout the prescribed durations. Additionally, because the removable garment is configured to be selectively worn, in implementations, the garment can include one or more retention features for assisting with positioning and securing the adhesive portions of the device to the torso of the patient.

In implementations, the patient-worn arrhythmia monitoring and treatment device further includes a patient notification output. In response to detecting one or more treatable arrhythmia conditions, the processor 118 is configured to prompt the patient for a response by issuing the patient notification output, which may be an audible output, tactile output, visual output, or some combination of any and all of these types of notification outputs. In the absence of a response to the notification output from the patient, the processor is configured to cause the therapy delivery circuit 130 to deliver the one or more therapeutic pulses to the patient.

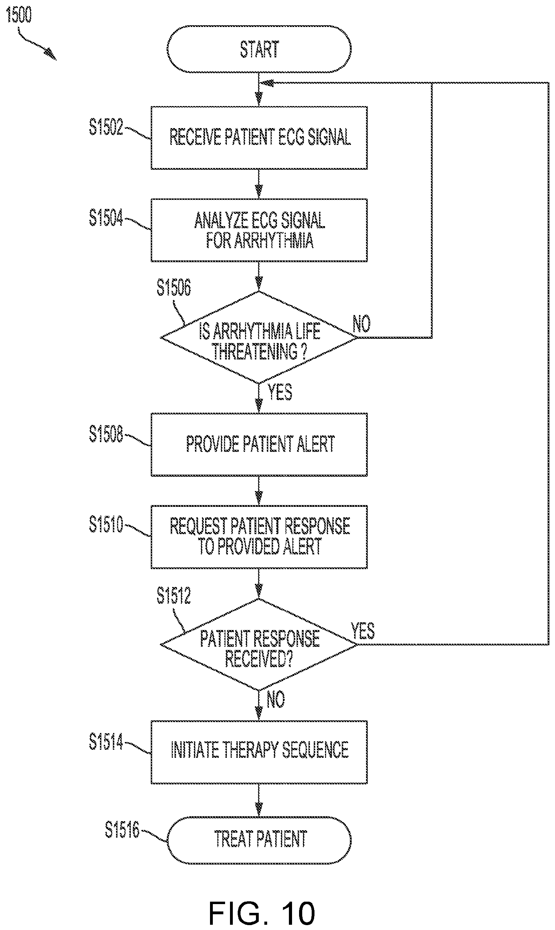

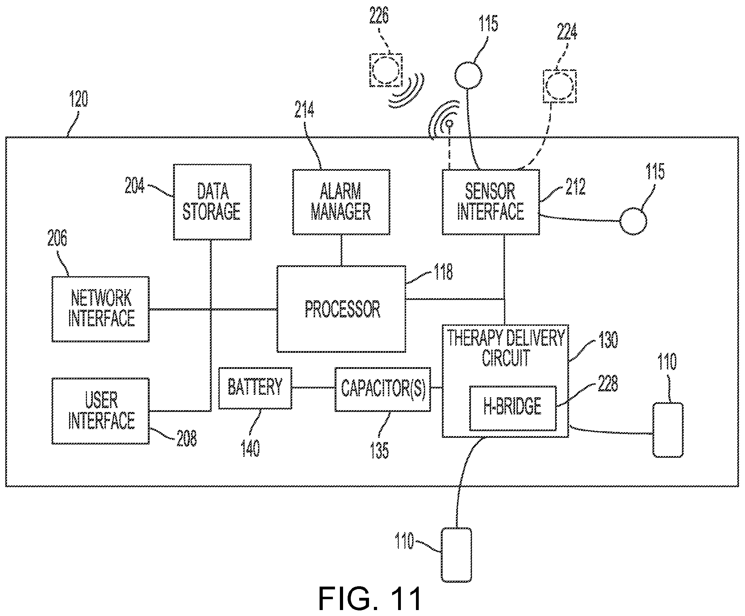

FIG. 10 depicts an example of a process 1500 for determining whether to initiate a therapy sequence and apply a therapeutic pulse to the body of a patient. In implementations, the processor 118, receives S1502 a patient ECG signal from the ECG electrodes 115 and analyzes S1504 the ECG signal for an arrhythmia condition. The processor 118 determines S1506 whether the arrhythmia is life threatening condition and requires treatment. If the arrhythmia is not life threatening, the processor 118 can cause a portion of the ECG signal to be stored in memory for later analysis and continue to monitor the patient ECG signal. If the arrhythmia is life threatening, the processor provides S1508 a patient notification output and requests S1510 a patient response to the provided notification output. In implementations, the patient responds to an alert by interacting with a user interface (e.g., the user interface 208 of FIG. 11), which includes, for example, one or more buttons (e.g. button 111 of FIG. 2C) or touch screen interface buttons with haptic feedback (e.g., touch screen buttons of the user interface of the device 10, 100 or remote devices, such as smartphones running user-facing interactive applications configured to communicate with the device 10, 100). The response may be, for example, pressing one or more buttons in a particular sequence or for a particular duration. The processor 118 determines S1512 whether the patient response was received. If the patient responds to the notification output, the processor 118 is notified that the patient is conscious and returns to a monitoring mode. If the patient is unconscious and unable to respond to the provided alert, the processor 118 initiates S 1514 the therapy sequence and treats S1516 the patient with the delivery of energy to the body of the patient.

In implementations, an example of a patient- worn arrhythmia monitoring and treatment device can include a short-term continuous monitoring defibrillator and/or pacing device, for example, a short-term outpatient wearable defibrillator. For example, such a short term outpatient wearable defibrillator can be prescribed by a physician for patients presenting with syncope. A wearable defibrillator can be configured to monitor patients presenting with syncope by, e.g., analyzing the patient's cardiac activity for aberrant patterns that can indicate abnormal physiological function. For example, such aberrant patterns can occur prior to, during, or after the onset of symptoms. In such an example implementation of the short-term wearable defibrillator, the electrode assembly can be adhesively attached to the patient's skin.

Example physiological sensors of a patient- worn arrhythmia monitoring and treatment device can include ECG sensing electrodes including a metal electrode with an oxide coating such as tantalum pentoxide electrodes, as described in, for example, U.S. Patent No. 6,253,099 entitled "Cardiac Monitoring Electrode Apparatus and Method".

In examples, the physiological sensors can include a heart rate sensor for detecting heart beats and monitoring the heart rate of the patient. For instance, such heart rate sensors can include the ECG sensing electrodes and associated circuitry described previously herein. In some examples, the heart rate sensors can include a radio frequency based pulse detection sensor or a pulse oximetry sensor worn adjacent an artery of the patient. In implementations, the heart rate sensor can be worn about the wrist of a patient, for example, incorporated on and/or within a watch or a bracelet. In some examples, the heart rate sensor can be integrated within a patch adhesively coupled to the skin of the patient over an artery.

In some examples, the therapy electrodes 110 can also be configured to include sensors configured to detect ECG signals as well as other physiological signals of the patient. The ECG data acquisition and conditioning circuitry 125 is configured to amplify, filter, and digitize these cardiac signals. One or more of the therapy electrodes 110 can be configured to deliver one or more therapeutic defibrillating shocks to the body of the patient when the medical device determines that such treatment is warranted based on the signals detected by the ECG sensing electrodes 115 and processed by the processor 118. Example therapy electrodes 110 can include conductive metal electrodes such as stainless steel electrodes that include, in certain implementations, one or more conductive gel deployment devices configured to deliver conductive gel to the metal electrode prior to delivery of a therapeutic shock.

In some implementations, patient-worn arrhythmia monitoring and treatment devices as described herein can be configured to switch between a therapeutic medical device and a monitoring medical device that is configured to only monitor a patient (e.g., not provide or perform any therapeutic functions). The therapeutic elements can be deactivated (e.g., by means or a physical or a software switch), essentially rendering the therapeutic medical device as a monitoring medical device for a specific physiologic purpose or a particular patient. As an example of a software switch, an authorized person can access a protected user interface of the medical device and select a preconfigured option or perform some other user action via the user interface to deactivate the therapeutic elements of the medical device.

FIG. 11 illustrates an example component-level view of the patient-worn arrhythmia monitoring and treatment device. As shown in FIG. 11, the medical device housing 120 can include a therapy delivery circuit 130 including a polarity switching component such as an H-bridge 228, a data storage 204, a network interface 206, a user interface 208 at least one battery 140, a sensor interface 212 that includes, for example, an ECG data acquisition and conditioning circuit 125, an alarm manager 214, least one processor 118, and one or more capacitors 135. A patient monitoring medical device can include components like those described with regard to FIG. 11, but does not include the therapy delivery circuit 130. Alternatively, a patient-worn monitoring device can include components like those described with regard to FIG. 11, but includes a switching mechanism for rendering the therapy delivery circuit 130 inoperative.

The therapy delivery circuit 130 is coupled to two or more therapy electrodes 110 configured to provide therapy to the patient. As indicated in FIG. 11, in examples, at least one of the two or more therapy electrodes 110 is within the housing 120 and another of the two or more therapy electrodes 110 is remote from the housing 120. For example, the therapy delivery circuit 130 includes, or is operably connected to, circuitry components that are configured to generate and provide the therapeutic shock. The circuitry components include, for example, resistors, one or more capacitors, relays and/or switches, an electrical bridge such as an H-bridge 228 (e.g., an H-bridge including a plurality of insulated gate bipolar transistors or IGBTs that deliver and truncate a therapy pulse), voltage and/or current measuring components, and other similar circuitry arranged and connected such that the circuitry work in concert with the therapy delivery circuit and under control of one or more processors (e.g., processor 118) to provide, for example, one or more pacing or defibrillation therapeutic pulses.

Pacing pulses can be used to treat cardiac arrhythmias such as bradycardia (e.g., in some implementations, less than 30 beats per minute) and tachycardia (e.g., in some implementations, more than 150 beats per minute) using, for example, fixed rate pacing, demand pacing, anti-tachycardia pacing, and the like. Defibrillation pulses can be used to treat ventricular tachycardia and/or ventricular fibrillation.

In implementations, each of the therapy electrodes 110 has a conductive surface adapted for placement adjacent the patient's skin and has an impedance reducing means contained therein or thereon for reducing the impedance between a therapy electrode and the patient's skin. Each of the therapy electrodes can include a conductive impedance reducing adhesive layer, such as a breathable anisotropic conductive hydrogel disposed between the therapy electrodes and the torso of the patient. In implementations, the adhesively coupled patient- worn arrhythmia monitoring and treatment device 10, 100 may include gel deployment circuitry configured to cause the delivery of conductive gel substantially proximate to a treatment site (e.g., a surface of the patient's skin in contact with the therapy electrode 110) prior to delivering therapeutic shocks to the treatment site. As described in U.S. Patent No. 9,008,801, titled "WEARABLE THERAPUETIC DEVICE," issued on April 14, 2015 (hereinafter the "'801 Patent"), the gel deployment circuitry can be configured to cause the delivery of conductive gel immediately before delivery of the therapeutic shocks to the treatment site, or within a short time interval, for example, within about 1 second, 5 seconds, 10 seconds, 30 seconds, or one minute before delivery of the therapeutic shocks to the treatment site. Such gel deployment circuitry can be coupled to or integrated within a first assembly 102, a second assembly 107, and/or a third assembly of the device.

When a treatable cardiac condition is detected and no patient response is received after device prompting, the gel deployment circuitry can be signaled to deploy the conductive gel. In some examples, the gel deployment circuitry can be constructed as one or more separate and independent gel deployment modules. Such modules can be configured to receive removable and/or replaceable gel cartridges (e.g., cartridges that contain one or more conductive gel reservoirs). As such, the gel deployment circuitry can be permanently disposed in the device as part of the therapy delivery systems, while the cartridges can be removable and/or replaceable.

In some implementations, the gel deployment modules can be implemented as gel deployment packs and include at least a portion of the gel deployment circuitry along with one or more gel reservoirs within the gel deployment pack. In such implementations, the gel deployment pack, including the one or more gel reservoirs and associated gel deployment circuitry can be removable and/or replaceable. In some examples, the gel deployment pack, including the one or more gel reservoirs and associated gel deployment circuitry, and the therapy electrode can be integrated into a therapy electrode assembly that can be removed and replaced as a single unit either after use, or if damaged or broken.

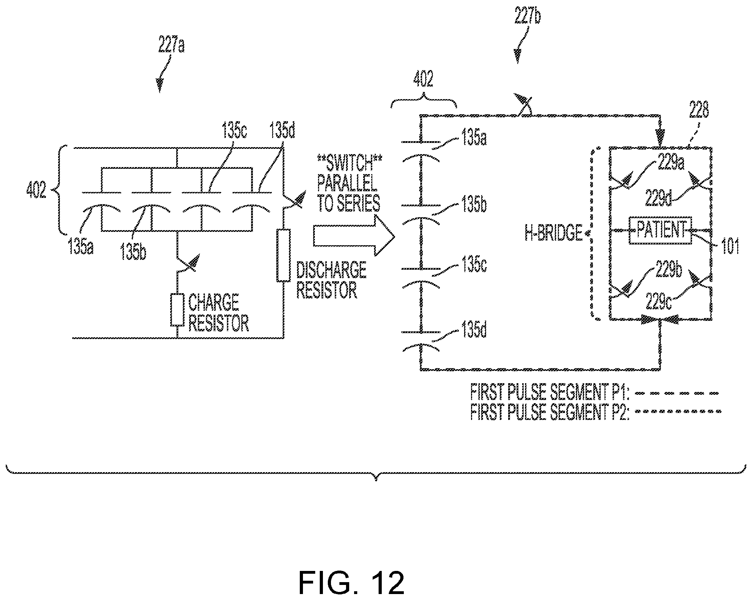

Continuing with the description of the example medical device of FIG. 11, in implementations, the one or more capacitors 135 is a plurality of capacitors (e.g., two, three, four or more capacitors) comprising a capacitor bank 402, as shown in FIG. 12. These capacitors 135 can be switched into a series connection during discharge for a defibrillation pulse. For example, four capacitors of approximately 650 µF can be used. In one implementation, the capacitors can have between 200 to 2500 volt surge rating and can be charged in approximately 5 to 30 seconds from a battery 140 depending on the amount of energy to be delivered to the patient.

For example, each defibrillation pulse can deliver between 60 to 400 joules (J) of energy. In some implementations, the defibrillating pulse can be a biphasic truncated exponential waveform, whereby the signal can switch between a positive and a negative portion (e.g., charge directions). An amplitude and a width of the two phases of the energy waveform can be automatically adjusted to deliver a predetermined energy amount.

The data storage 204 can include one or more of non-transitory computer readable media, such as flash memory, solid state memory, magnetic memory, optical memory, cache memory, combinations thereof, and others. The data storage 204 can be configured to store executable instructions and data used for operation of the patient-worn arrhythmia monitoring and treatment device. In certain implementations, the data storage 204 can include executable instructions that, when executed, are configured to cause the processor 118 to perform one or more functions.

In some examples, the network interface 206 can facilitate the communication of information between the patient-worn arrhythmia monitoring and treatment device and one or more other devices or entities over a communications network. For example, the network interface 206 can be configured to communicate with a remote computing device such as a remote server or other similar computing device. The network interface 206 can include communications circuitry for transmitting data in accordance with a BLUETOOTH wireless standard for exchanging such data over short distances to an intermediary device(s) (e.g., a base station, a "hotspot" device, a smartphone, a tablet, a portable computing device, and/or other devices in proximity of the wearable medical device 100). The intermediary device(s) may in turn communicate the data to a remote server over a broadband cellular network communications link. The communications link may implement broadband cellular technology (e.g., 2.5G, 2.75G, 3G, 4G, 5G cellular standards) and/or Long-Term Evolution (LTE) technology or GSM/EDGE and UMTS/HSPA technologies for high-speed wireless communication. In some implementations, the intermediary device(s) may communicate with a remote server over a WI-FI communications link based on the IEEE 802.11 standard.

In certain implementations, the user interface 208 can include one or more physical interface devices such as input devices, output devices, and combination input/output devices and a software stack configured to drive operation of the devices. These user interface elements may render visual, audio, and/or tactile content. Thus, the user interface 208 may receive input or provide output, thereby enabling a user to interact with the medical device. In some implementations, the user interface 208 can be implanted as a hand-held user interface device. (See, for example, the patient interface pod 40 of FIG. 1.) For instance, the hand-held user interface device can be a smartphone or other portable device configured to communicate with the processor 118 via the network interface 206. In an implementation, the hand-held user interface device may also be the intermediary device for facilitating the transfer of information from the device to a remote server.

As described, the patient-worn arrhythmia monitoring and treatment device can also include at least one battery 140 configured to provide power to one or more components, such as the one or more capacitors 135. The battery 140 can include a rechargeable multi-cell battery pack. In one example implementation, the battery 140 can include three or more 2200 mAh lithium ion cells that provide electrical power to the other device components. For example, the battery 140 can provide its power output in a range of between 20 mA to 1000 mA (e.g., 40 mA) output and can support 24 hours, 48 hours, 72 hours, or more, of runtime between charges. As previously descried in detail, in certain implementations, the battery capacity, runtime, and type (e.g., lithium ion, nickel-cadmium, or nickel-metal hydride) can be changed to best fit the specific application of the medical device.

The sensor interface 212 can be coupled to one or more sensors configured to monitor one or more physiological parameters of the patient. As shown in FIG. 11, the sensors can be coupled to the medical device controller (e.g., processor 118) via a wired or wireless connection. The sensors can include one or more sensing electrodes (e.g., ECG sensing electrodes 115), vibrations sensors 224, and tissue fluid monitors 226 (e.g., based on ultra-wide band radiofrequency devices). For example, the sensor interface 212 can include ECG circuitry (such as ECG acquisition and conditioning circuitry 125 of FIG. 3) and/or accelerometer circuitry, which are each configured to receive and condition the respective sensor signals.

The sensing electrodes can monitor, for example, a patient's ECG information. For example, the sensing electrodes of FIG. 11 can be ECG sensing electrodes 115 and can include conductive electrodes with stored gel deployment (e.g., metallic electrodes with stored conductive gel configured to be dispersed in the electrode-skin interface when needed), conductive electrodes with a conductive adhesive layer, or dry electrodes (e.g., a metallic substrate with an oxide layer in direct contact with the patient's skin). The ECG sensing electrodes 115 can be configured to measure the patient's ECG signals. The ECG sensing electrodes 115 can transmit information descriptive of the ECG signals to the sensor interface 212 for subsequent analysis.

The vibrational sensors 224 can detect a patient's cardiac or pulmonary (cardiopulmonary) vibration information. For example, the cardiopulmonary vibrations sensors 224 can be configured to detect cardio-vibrational biomarkers in a cardio-vibrational signal, including any one or all of S1, S2, S3, and S4 cardio-vibrational biomarkers. From these cardio-vibrational biomarkers, certain electromechanical metrics can be calculated, including any one or more of electromechanical activation time (EMAT), percentage of EMAT (% EMAT), systolic dysfunction index (SDI), left ventricular diastolic perfusion time (LDPT), and left ventricular systolic time (LVST). The cardiopulmonary vibrations sensors 224 may also be configured to detect hear wall motion, for example, by placement of the sensor 224 in the region of the apical beat.

The vibrations sensors 224 can include an acoustic sensor configured to detect vibrations from a subject's cardiac or pulmonary (cardiopulmonary) system and provide an output signal responsive to the detected vibrations of the targeted organ. For instance, in some implementations, the vibrations sensors 224 are able to detect vibrations generated in the trachea or lungs due to the flow of air during breathing. The vibrations sensors 224 can also include a multi-channel accelerometer, for example, a three channel accelerometer configured to sense movement in each of three orthogonal axes such that patient movement/body position can be detected. The vibrations sensors 224 can transmit information descriptive of the cardiopulmonary vibrations information or patient position/movement to the sensor interface 212 for subsequent analysis.

The tissue fluid monitors 226 can use radio frequency (RF) based techniques to assess changes of accumulated fluid levels over time. For example, the tissue fluid monitors 226 can be configured to measure fluid content in the lungs (e.g., time-varying changes and absolute levels), for diagnosis and follow-up of pulmonary edema or lung congestion in heart failure patients. The tissue fluid monitors 226 can include one or more antennas configured to direct RF waves through a patient's tissue and measure output RF signals in response to the waves that have passed through the tissue. In certain implementations, the output RF signals include parameters indicative of a fluid level in the patient's tissue. The tissue fluid monitors 226 can transmit information descriptive of the tissue fluid levels to the sensor interface 212 for subsequent analysis.

The sensor interface 212 can be coupled to any one or combination of sensing electrodes/other sensors to receive other patient data indicative of patient parameters. Once data from the sensors has been received by the sensor interface 212, the data can be directed by the processor 118 to an appropriate component within the medical device. For example, if cardiac data is collected by the cardiopulmonary vibrations sensor 224 and transmitted to the sensor interface 212, the sensor interface 212 can transmit the data to the processor 118 which, in turn, relays the data to a cardiac event detector. The cardiac event data can also be stored on the data storage 204.

An alarm manager 214 can be configured to manage alarm profiles and notify one or more intended recipients of events specified within the alarm profiles as being of interest to the intended recipients. These intended recipients can include external entities such as users (e.g., patients, physicians, other caregivers, patient care representatives, and other authorized monitoring personnel) as well as computer systems (e.g., monitoring systems or emergency response systems). The alarm manager 214 can be implemented using hardware or a combination of hardware and software. For instance, in some examples, the alarm manager 214 can be implemented as a software component that is stored within the data storage 204 and executed by the processor 118. In this example, the instructions included in the alarm manager 214 can cause the processor 118 to configure alarm profiles and notify intended recipients according to the configured alarm profiles. In some examples, alarm manager 214 can be an application-specific integrated circuit (ASIC) that is coupled to the processor 118 and configured to manage alarm profiles and notify intended recipients using alarms specified within the alarm profiles. Thus, examples of alarm manager 214 are not limited to a particular hardware or software implementation.

In some implementations, the processor 118 includes one or more processors (or one or more processor cores) that each are configured to perform a series of instructions that result in manipulated data and/or control the operation of the other components of the patient-worn arrhythmia monitoring and treatment device. In some implementations, when executing a specific process (e.g., cardiac monitoring), the processor 118 can be configured to make specific logic-based determinations based on input data received, and be further configured to provide one or more outputs that can be used to control or otherwise inform subsequent processing to be carried out by the processor 118 and/or other processors or circuitry with which processor 118 is communicatively coupled. Thus, the processor 118 reacts to a specific input stimulus in a specific way and generates a corresponding output based on that input stimulus. In some example cases, the processor 118 can proceed through a sequence of logical transitions in which various internal register states and/or other bit cell states internal or external to the processor 118 can be set to logic high or logic low. The processor 118 can be configured to execute a function stored in software. For example, such software can be stored in a data store coupled to the processor 118 and configured to cause the processor 118 to proceed through a sequence of various logic decisions that result in the function being executed. The various components that are described herein as being executable by the processor 118 can be implemented in various forms of specialized hardware, software, or a combination thereof. For example, the processor can be a digital signal processor (DSP) such as a 24-bit DSP processor. The processor 118 can be a multi-core processor, e.g., a processor having two or more processing cores. The processor can be an Advanced RISC Machine (ARM) processor such as a 32-bit ARM processor or a 64-bit ARM processor. The processor can execute an embedded operating system and include services provided by the operating system that can be used for file system manipulation, display & audio generation, basic networking, firewalling, data encryption and communications.

In implementations, the therapy delivery circuit 130 includes, or is operably connected to, circuitry components that are configured to generate and provide the therapeutic shock. As described previously, the circuitry components include, for example, resistors, one or more capacitors 135, relays and/or switches, an electrical bridge such as an H-bridge 228 (e.g., an H-bridge circuit including a plurality of switches, (e.g. insulated gate bipolar transistors or IGBTs, silicon carbide field effect transistors (SiC FETs), metal-oxide semiconductor field effect transistors (MOSFETS), silicon-controlled rectifiers (SCRs), or other high current switching devices)), voltage and/or current measuring components, and other similar circuitry components arranged and connected such that the circuitry components work in concert with the therapy delivery circuit 130 and under control of one or more processors (e.g., processor 118) to provide, for example, one or more pacing or defibrillation therapeutic pulses.

In implementations, the patient- worn arrhythmia monitoring and treatment device 10, 100 further includes a source of electrical energy, for example, the one or more capacitors 135, that stores and provides energy to the therapy delivery circuit 130. The one or more therapeutic pulses are defibrillation pulses of electrical energy, and the one or more treatable arrhythmias include ventricular fibrillation and ventricular tachycardia. In implementations, the one or more therapeutic pulses are biphasic exponential pulses. Such therapeutic pulses can be generated by charging the one or more capacitors 135 and discharging the energy stored in the one or more capacitors 135 into the patient via the plurality of therapy electrodes. For example, the therapy delivery circuit 130 can include one or more power converters for controlling the charging and discharging of the one or more capacitors 135. In some implementations, the discharge of energy from the one or more capacitors 135 can be controlled by, for example, an H-bridge that controls the discharge of energy into the body of the patient, like the H-bridge circuit described in U.S. Patent No. 6,280,461, titled "PATIENT- WORN ENERGY DELIVERY APPARATUS," issued on August 28, 2001, and U.S. Patent No. 8,909,335, titled "METHOD AND APPARATUS FOR APPLYING A RECTILINEAR BIPHASIC POWER WAVEFORM TO A LOAD," issued on December 9, 2014.

As shown in the embodiment to FIG. 12, the H-bridge 228 is electrically coupled to a capacitor bank 402 including four capacitors 135a-d that are charged in parallel at a preparation phase 227a and discharged in series at a treatment phase 227b. In some implementations, the capacitor bank 402 can include more or fewer than four capacitors 135. During the treatment phase 227b, the H-bridge 228 applies a therapeutic pulse that causes current to flow through the torso 5 of the patient in desired directions for desired durations. The H-bridge 228 includes H-bridge switches 229a-d that are opened and closed selectively by a switching transistor such as insulated gate bipolar transistors (IGBTs), silicon carbide field effect transistors (SiC FETs), metal-oxide semiconductor field effect transistors (MOSFETS), silicon-controlled rectifiers (SCRs), or other high current switching devices. Switching a pair of transistors to a closed position, for example switches 229a and 229c, enables current to flow in a first direction for first pulse segment PL Opening switches 229a and 229c and closing switches 229b and 229d enables current to flow through the torso 5 of the patient in a second pulse segment P2 directionally opposite the flow of the first pulse segment P1.

Although the subject matter contained herein has been described in detail for the purpose of illustration, it is to be understood that such detail is solely for that purpose and that the present disclosure is not limited to the disclosed embodiments, but, on the contrary, is intended to cover modifications and equivalent arrangements that are within the scope of this description. For example, it is to be understood that the present disclosure contemplates that, to the extent possible, one or more features of any embodiment can be combined with one or more features of any other embodiment.

Other examples are within the scope of the disclosure. Additionally, certain functions described above can be implemented using software, hardware, firmware, hardwiring, or combinations of any of these. Features implementing functions can also be physically located at various positions, including being distributed such that portions of functions are implemented at different physical locations. The scope of protection is defined solely by the appended claims.

## Claims

1. A patient-worn arrhythmia monitoring and treatment device for continuous wear during daily activities, comprising:
at least two pads (105, 109), comprising
an adhesive layer configured to affix each one of the at least two pads to skin on a torso (5) of a patient,
at least one of a pair of sensing electrodes (115) disposed on each one of the at least two pads, the pair of sensing electrodes configured to sense surface ECG activity of the patient;
at least one of a pair of therapy electrodes (110) disposed on each one of the at least two pads, the pair of therapy electrodes configured to deliver one or more therapeutic pulses to the patient, and
at least one housing (120) coupled to one of the at least two pads, wherein the at least one housing 120 forms a watertight seal with the one pad of the at least two pads,
wherein the one pad includes one or more receptacles (106) for receiving the at least one housing in a watertight mating,
a controller (20) disposed within the at least one housing, coupled to the one of the at least two pads and in communication with the pairs of sensing and therapy electrodes, wherein the controller (20) comprises a source of electrical energy, a processor, ECG acquisition and conditioning circuitry and high-voltage therapy control circuitry disposed within the housing, and wherein the controller is configured to monitor for cardiac arrhythmias based on the sensed surface ECG activity of the patient and cause the delivery of the one or more therapeutic pulses to the patient; and
a removable garment (51, 53) configured to be worn about the torso of the patient during a prescribed period of 1 day or longer to immobilize on the torso the one of the at least two pads to which the controller is coupled thereby offsetting sheer forces during physical activity of the patient, and
wherein the removable garment comprises a compression portion configured to immobilize the removable garment relative to a skin surface of the thoracic region of the patient, and all of the removable garment is manufactured from a stretchable fabric, or wherein the compression portion comprises an elasticized panel disposed in the removable garment.

2. The device of claim 1,
wherein the removable garment is a wearable support (51, 53) configured to at least partially support a weight of the patient-worn arrhythmia monitoring and treatment device, the removable wearable support comprising
a fabric encircling a torso of a patient, wherein the removable wearable support comprises a compression portion configured to immobilize the removable wearable support relative to a skin surface of the thoracic region of the patient, and wherein the compression portion comprises at least one of elasticized thread, an elasticized panel, or an adjustable tension element; and
an engagement surface disposed on one side of the fabric, the engagement surface configured to engage immovably with at least one of two pads (105, 109) configured to be affixed to an upper torso (5) of the patient, the at least one of two pads comprising an adhesive layer configured to affix the at least one of two pads to skin on the upper torso of the patient,
wherein the housing is configured to engage with the engagement surface of the removable wearable support.

3. The device of claim 1 or claim 2, wherein the at least two pads (105, 109) are configured for continuous wear for durations of at least one of a day, one week, two weeks, a month, six months, and one year.

4. The device of any preceding claim, wherein facilitation of continuous wear comprises one or both of:
each one of the at least two pads (105, 109) comprising a breathable hydrogel layer disposed between the patient's skin and each of the pairs of sensing and therapy electrodes; and
the adhesive layer comprising a breathable adhesive material.

5. The device of any preceding claim, wherein each one of the at least two pads (105, 109) comprises waterproof or water-resistant material to prevent ingress of liquid between the patient's skin and each one of the at least two pads to facilitate the continuous wear at least during periods of bathing or showering.

6. The device of claim 1 or 2, wherein the removable garment (51, 53) is configured for selective wear during certain activities of finite duration including at least one of athletic activities, showering, bathing, and sleeping, and optionally comprises a retention feature configured to facilitate the selective wear during certain activities by allowing separation of the removable garment from the one of the at least two pads (105, 109) to which the controller (20) is coupled.

7. The device of claim 1 or claim 2, wherein a first of the at least two pads (105, 109) has a first weight and a second of the at least two pads has a second weight, wherein
the first weight is greater than the second weight;
wherein the removable garment (51, 53) is optionally configured to at least partially support the weight of the first of the at least two pads, wherein the first of the at least two pads optionally weighs between 1kg-2.5kg and the second of the at least two pads optionally weighs between 0.5kg-1kg, and wherein the first of the at least two pads is the one of the at least two pads to which the controller is coupled..

8. The device of claim 1 or 2, wherein the removable garment (51, 53) is at least one of a shoulder strap, a vest, a shirt, a belt, a harness, a bandeau, and a sash.

9. The device of claim 1 or 2, wherein the removable garment (51, 53) comprises a closure mechanism (52) including at least one of a ratchet strap, an adjustable buckle, an extendable and moveable hook and loop fastener strip, a tie, a snap, and a button.

10. The device of claim 1 or 2, wherein immobilizing the one of the at least two pads (105, 109) to which the controller is coupled comprises at least partially supporting a weight of the one of the at least two pads to which the controller is coupled.

11. The device of claim 1 or 2, wherein the removable garment (51, 53) further comprises a retention feature for engaging with the housing.

12. The device of claim 11, wherein the retention feature comprises one or more of: a) a hole sized and shaped to accommodate the housing when inserted through the hole; b) a fastener including at least one of hook and loop fastener strip, a tie, a snap, and a button; c) an interlock disposed on an interior surface of the removable garment, the interlock configured to engage a corresponding mating portion disposed on an exterior surface of the housing; d) a non-slip surface disposed on an interior surface of the removable garment such that the removable garment remains stationary relative to the housing; or e) a conforming cup formed as non-removable portion of the removable garment such that the removable garment support remains stationary relative to the housing when the housing is received in the conforming cup.

## Patentansprüche

1. Vom Patienten getragene Vorrichtung zur Überwachung und Behandlung von Arrhythmien für das kontinuierliche Tragen während der täglichen Aktivitäten, umfassend:
mindestens zwei Pads (105, 109), umfassend:
eine Klebstoffschicht, die konfiguriert ist, um jedes der mindestens zwei Pads an der Haut eines Torsos (5) eines Patienten zu befestigen,
mindestens eine aus einem Paar von Erfassungselektroden (115), die auf jedem der mindestens zwei Pads angeordnet sind, wobei das Paar von Erfassungselektroden konfiguriert ist, um die Oberflächen-EKG-Aktivität des Patienten zu erfassen;
mindestens eine eines Paares von Therapieelektroden (110), die auf jedem der mindestens zwei Pads angeordnet sind, wobei das Paar von Therapieelektroden konfiguriert ist, um einen oder mehrere therapeutische Impulse an den Patienten abzugeben, und
mindestens ein Gehäuse (120), das mit einem der mindestens zwei Pads gekoppelt ist, wobei das mindestens eine Gehäuse (120) eine wasserdichte Dichtung mit dem einen Pad der mindestens zwei Pads bildet, wobei das eine Pad eine oder mehrere Aufnahmen (106) zum Empfangen des mindestens einen Gehäuses in einer wasserdichten Passung einschließt,
eine Steuerung (20), die innerhalb des mindestens einen Gehäuses angeordnet ist, mit dem einen der mindestens zwei Pads gekoppelt ist und mit den Paaren von Erfassungs- und Therapieelektroden in Verbindung steht, wobei die Steuerung (20) eine Quelle für elektrische Energie, einen Prozessor, eine EKG-Erfassungs- und - aufbereitungsschaltlogik und eine Hochspannungs-Therapiesteuerschaltlogik, die innerhalb des Gehäuses angeordnet sind, und wobei die Steuerung konfiguriert ist, um basierend auf der erfassten Oberflächen-EKG-Aktivität des Patienten Herzrhythmusstörungen zu überwachen und die Abgabe des einen oder der mehreren Therapieimpulse an den Patienten zu bewirken; und
ein entfernbares Kleidungsstück (51, 53), das konfiguriert ist, um während eines vorgeschriebenen Zeitraums von einem Tag oder länger um den Torso des Patienten getragen zu werden, um das eine der mindestens zwei Pads, mit denen die Steuerung gekoppelt ist, am Torso zu fixieren, wodurch Scherkräfte während der körperlichen Aktivität des Patienten ausgeglichen werden, und
wobei das entfernbare Kleidungsstück einen Kompressionsabschnitt umfasst, der konfiguriert ist, um das entfernbare Kleidungsstück relativ zu einer Hautoberfläche der Brustregion des Patienten zu fixieren, und das gesamte entfernbare Kleidungsstück aus einem dehnbaren Gewebe hergestellt ist, oder wobei der Kompressionsabschnitt ein elastisches Paneel umfasst, das in dem entfernbaren Kleidungsstück angeordnet ist.

2. Vorrichtung nach Anspruch 1,
wobei das entfernbare Kleidungsstück eine tragbare Stütze (51, 53) ist, die konfiguriert ist, um mindestens teilweise ein Gewicht der vom Patienten getragenen Vorrichtung zur Überwachung und Behandlung von Arrhythmien zu stützen, die entfernbare tragbare Stütze umfassend:
ein Gewebe, das einen Torso eines Patienten umgibt, wobei die entfernbare, tragbare Stütze einen Kompressionsabschnitt umfasst, der konfiguriert ist, um die entfernbare, tragbare Stütze relativ zu einer Hautoberfläche der Brustregion des Patienten zu fixieren, und wobei der Kompressionsabschnitt mindestens einen elastifizierten Faden, ein elastifiziertes Paneel oder ein einstellbares Spannelement umfasst; und
eine Eingriffsoberfläche, die auf einer Seite des Gewebes angeordnet ist, wobei die Eingriffsoberfläche konfiguriert ist, um unbeweglich mit mindestens einem von zwei Pads (105, 109) in Eingriff zu kommen, die konfiguriert sind, um an einem Torso (5) des Patienten befestigt zu werden, wobei das mindestens eine von zwei Pads eine Klebstoffschicht umfasst, die konfiguriert ist, um das mindestens eine von zwei Pads an der Haut auf dem Torso des Patienten zu befestigen,
wobei das Gehäuse konfiguriert ist, um mit der Eingriffsoberfläche der abnehmbaren tragbaren Stütze in Eingriff zu kommen.

3. Vorrichtung nach Anspruch 1 oder 2, wobei die mindestens zwei Pads (105, 109) konfiguriert sind, um kontinuierlich über einen Zeitraum von mindestens einem Tag, einer Woche, zwei Wochen, einem Monat, sechs Monaten und einem Jahr getragen zu werden.

4. Vorrichtung nach einem der vorstehenden Ansprüche, wobei die Erleichterung des kontinuierlichen Tragens eines oder beides von Folgendem umfasst:
jedes der mindestens zwei Pads (105, 109), umfassend eine atmungsaktive Hydrogelschicht, die zwischen der Haut des Patienten und jedem der Paare von Mess- und Therapieelektroden angeordnet ist; und
die Klebstoffschicht, umfassend ein atmungsaktives Klebstoffmaterial.

5. Vorrichtung nach einem der vorstehenden Ansprüche, wobei jedes der mindestens zwei Pads (105, 109) wasserdichtes oder wasserbeständiges Material umfasst, um das Eindringen von Flüssigkeit zwischen der Haut des Patienten und jedem der mindestens zwei Pads zu verhindern, um das kontinuierliche Tragen mindestens während der Zeiträume des Badens oder Duschens zu erleichtern.

6. Vorrichtung nach Anspruch 1 oder 2, wobei die abnehmbare Stütze oder das abnehmbare Kleidungsstück (51, 53) konfiguriert ist, um während bestimmter Aktivitäten von begrenzter Dauer selektiv getragen zu werden, einschließlich mindestens einer von sportlichen Aktivitäten, Duschen, Baden und Schlafen, und optional ein Rückhaltemerkmal umfasst, das konfiguriert ist, um das selektive Tragen während bestimmter Aktivitäten zu erleichtern, indem es die Trennung des abnehmbaren Kleidungsstücks von einem der mindestens zwei Polster (105, 109) ermöglicht, mit dem die Steuerung (20) gekoppelt ist.

7. Vorrichtung nach Anspruch 1 oder 2, wobei ein erstes der mindestens zwei Pads (105, 109) ein erstes Gewicht aufweist und ein zweites der mindestens zwei Pads ein zweites Gewicht aufweist, wobei
das erste Gewicht größer ist als das zweite Gewicht; wobei das abnehmbare Kleidungsstück (51, 53) optional konfiguriert ist, um mindestens teilweise das Gewicht der ersten der mindestens zwei Stützen zu tragen, wobei die erste der mindestens zwei Stützen optional zwischen 1 kg und 2,5 kg wiegt und die zweite der mindestens zwei Stützen optional zwischen 0,5 kg und 1 kg wiegt, und wobei die erste der mindestens zwei Stützen diejenige der mindestens zwei Stützen ist, mit der die Steuerung gekoppelt ist.

8. Vorrichtung nach Anspruch 1 oder 2, wobei das abnehmbare Kleidungsstück (51, 53) mindestens ein Schultergurt, eine Weste, ein Hemd, ein Gürtel, ein Geschirr, ein Bandeau oder eine Schärpe ist.

9. Vorrichtung nach Anspruch 1 oder 2, wobei das abnehmbare Kleidungsstück (51, 53) einen Verschlussmechanismus (52) umfasst, der mindestens eines von einem Ratschenband, einer verstellbaren Schnalle, einem ausziehbaren und beweglichen Klettverschlussstreifen, einem Binder, einem Druckknopf und einem Knopf einschließt.

10. Vorrichtung nach Anspruch 1 oder 2, wobei das Immobilisieren der einen der mindestens zwei Stützen (105, 109), an die die Steuerung gekoppelt ist, mindestens das teilweise Unterstützen eines Gewichts der einen der mindestens zwei Stützen umfasst, an die die Steuerung gekoppelt ist.

11. Vorrichtung nach Anspruch 1 oder 2, wobei das abnehmbare Kleidungsstück (51, 53) ferner ein Rückhaltemerkmal für den Eingriff mit dem Gehäuse umfasst.

12. Vorrichtung nach Anspruch 11, wobei das Rückhaltemerkmal eines oder mehreres von Folgendem umfasst: a) ein Loch, das so bemessen und geformt ist, dass es das Gehäuse aufnimmt, wenn es durch das Loch eingeführt wird; b) einen Befestiger, der mindestens einen von Klettverschlussstreifen, einem Band, einem Schnappverschluss und einem Knopf umfasst; c) eine Verriegelung, die auf einer Innenoberfläche des abnehmbaren Kleidungsstücks angeordnet ist, wobei die Verriegelung konfiguriert ist, um in einen entsprechenden passenden Abschnitt einzugreifen, der auf einer Außenoberfläche des Gehäuses angeordnet ist; d) eine rutschfeste Oberfläche, die auf einer Innenoberfläche des abnehmbaren Kleidungsstücks angeordnet ist, sodass das abnehmbare Kleidungsstück relativ zu dem Gehäuse stationär bleibt; oder e) eine anpassungsfähige Schale, die als nicht entfernbarer Abschnitt des entfernbaren Kleidungsstücks ausgebildet ist, sodass die Stütze für das entfernbare Kleidungsstück relativ zum Gehäuse stationär bleibt, wenn das Gehäuse in der anpassungsfähigen Schale empfangen wird.

## Revendications

1. Dispositif de surveillance et de traitement de l'arythmie porté par le patient pour un port continu pendant les activités quotidiennes, comprenant :
au moins deux tampons (105, 109), comprenant une couche adhésive configurée pour fixer chacun des au moins deux tampons à la peau d'un torse (5) d'un patient, au moins une d'une paire d'électrodes de détection (115) disposées sur chacun des au moins deux tampons, la paire d'électrodes de détection étant configurée pour détecter l'activité ECG de surface du patient ;
au moins l'une d'une paire d'électrodes de thérapie (110) disposées sur chacun des au moins deux tampons, la paire d'électrodes de thérapie étant configurée pour délivrer une ou plusieurs impulsions thérapeutiques au patient, et
au moins un boîtier (120) couplé à l'un des au moins deux tampons, dans lequel l'au moins un boîtier (120) forme un joint étanche avec le tampon des au moins deux tampons dans lequel le tampon comporte un ou plusieurs réceptacles (106) pour recevoir l'au moins un boîtier dans un accouplement étanche,
un dispositif de commande (20) disposé dans l'au moins un boîtier, couplé à l'un des au moins deux tampons et en communication avec les paires d'électrodes de détection et de thérapie, dans lequel le dispositif de commande (20) comprend une source d'énergie électrique, un processeur, un circuit d'acquisition et de conditionnement ECG et un circuit de commande de thérapie haute tension disposés dans le boîtier, et dans lequel le dispositif de commande est configuré pour surveiller les arythmies cardiaques sur la base de l'activité ECG de surface détectée du patient et provoquer l'administration des une ou plusieurs des impulsions thérapeutiques au patient ; et
un vêtement amovible (51, 53) configuré pour être porté autour du torse du patient pendant une période prescrite d'un jour ou plus pour immobiliser sur le torse l'un des au moins deux tampons auxquels le dispositif de commande est couplé compensant ainsi les forces de cisaillement pendant l'activité physique du patient, et
dans lequel le vêtement amovible comprend une partie de compression configurée pour immobiliser le vêtement amovible par rapport à une surface de peau de la région thoracique du patient, et la totalité du vêtement amovible est fabriquée à partir d'un tissu extensible, ou dans lequel la partie de compression comprend un panneau élastique disposé dans le vêtement amovible.

2. Dispositif selon la revendication 1,
dans lequel le vêtement amovible est un support portable (51, 53) configuré pour supporter au moins partiellement un poids du dispositif de surveillance et de traitement de l'arythmie porté par le patient, le support portable amovible comprenant
un tissu entourant le torse d'un patient, dans lequel le support portable amovible comprend une partie de compression configurée pour immobiliser le support portable amovible par rapport à une surface de peau de la région thoracique du patient, et dans lequel la partie de compression comprend au moins l'un d'un fil élastique, d'un panneau élastique, ou d'un élément de tension réglable ; et
une surface de contact disposée sur un côté du tissu, la surface de contact étant configurée pour entrer en contact de manière immobile avec au moins l'un de deux tampons (105, 109) configurés pour être fixés à un torse supérieur (5) du patient, l'au moins un des deux tampons comprenant une couche adhésive configurée pour fixer l'au moins un de deux tampons sur la peau du torse supérieur du patient,
dans lequel le boîtier est configuré pour entrer en contact avec la surface de contact du support portable amovible.

3. Dispositif selon la revendication 1 ou la revendication 2, dans lequel les au moins deux tampons (105, 109) sont configurés pour un port continu pendant une durée d'au moins l'une des périodes suivantes : une journée, une semaine, deux semaines, un mois, six mois, et un an.

4. Dispositif selon une quelconque revendication précédente, dans lequel la facilitation du port continu comprend l'un ou les deux éléments suivants :
chacun des au moins deux tampons (105, 109) comprenant une couche d'hydrogel respirant disposée entre la peau du patient et chacune des paires d'électrodes de détection et de thérapie ; et
la couche adhésive comprenant un matériau adhésif respirant.

5. Dispositif selon une quelconque revendication précédente, dans lequel chacun des au moins deux tampons (105, 109) comprend un matériau imperméable ou résistant à l'eau pour empêcher la pénétration de liquide entre la peau du patient et chacun des au moins deux tampons pour faciliter le port continu au moins pendant les périodes de bain ou de douche.

6. Dispositif selon la revendication 1 ou 2, dans lequel le support ou vêtement amovible (51, 53) est configuré pour un port sélectif pendant certaines activités à durée limitée comportant au moins une activité parmi les activités sportives, la douche, le bain, et le sommeil, et comprend éventuellement une fonction de rétention configurée pour faciliter le port sélectif pendant certaines activités en permettant la séparation du vêtement amovible de l'un des au moins deux tampons (105, 109) auquel le dispositif de commande (20) est couplé.

7. Dispositif selon la revendication 1 ou la revendication 2, dans lequel un premier des au moins deux tampons (105, 109) a un premier poids et un second des au moins deux tampons a un second poids, dans lequel le premier poids est supérieur au second poids ;
dans lequel le vêtement amovible (51, 53) est éventuellement configuré pour supporter au moins partiellement le poids du premier des au moins deux tampons, dans lequel le premier des au moins deux tampons pèse éventuellement entre 1 kg et 2,5 kg et le second des au moins deux tampons pèse éventuellement entre 0,5 kg et 1 kg, et dans lequel le premier des au moins deux tampons est celui des au moins deux tampons auquel le dispositif de commande est couplé.

8. Dispositif selon la revendication 1 ou 2, dans lequel le vêtement amovible (51, 53) est au moins l'un d'une bandoulière, d'un gilet, d'une chemise, d'une ceinture, d'un harnais, d'un bandeau, et d'une écharpe.

9. Dispositif selon la revendication 1 ou 2, dans lequel le vêtement amovible (51, 53) comprend un mécanisme de fermeture (52) comportant au moins l'un élément parmi : une sangle à cliquet, une boucle réglable, une bande de fermeture auto-agrippante extensible et mobile, une attache, un bouton-pression, et un bouton.

10. Dispositif selon la revendication 1 ou 2, dans lequel l'immobilisation de l'un des au moins deux tampons (105, 109) auquel le dispositif de commande est couplé comprend le support au moins partiel d'un poids de l'un des au moins deux tampons auquel le dispositif de commande est couplé.

11. Dispositif selon la revendication 1 ou 2, dans lequel le vêtement amovible (51, 53) comprend également un élément de retenue destiné à entrer en contact avec le boîtier.

12. Dispositif selon la revendication 11, dans lequel la fonction de rétention comprend un ou plusieurs éléments parmi : a) un trou dimensionné et formé pour accueillir le boîtier lorsqu'il est inséré à travers le trou ; b) une attache comportant au moins une bande de fermeture auto-agrippante, une attache, un bouton-pression, et un bouton ; c) un verrouillage disposé sur une surface intérieure du vêtement amovible, le verrouillage étant configuré pour entrer en contact avec une partie d'accouplement correspondante disposée sur une surface extérieure du boîtier ; d) une surface antidérapante disposée sur une surface intérieure du vêtement amovible de sorte que le vêtement amovible reste immobile par rapport au boîtier ; ou e) une coupelle conformée formée comme partie non amovible du vêtement amovible de sorte que le support de vêtement amovible reste immobile par rapport au boîtier lorsque le boîtier est reçu dans la coupelle conformée.
